# EUROPEAN PATENT APPLICATION

(11) **EP 4 685 149 A1**
(43) Date of publication of application: **28.01.2026**
(21) Application number: 24775281.9
(22) Date of filing: 21.03.2024
(51) Int. Cl.: C07H 19/16, C07D 473/34, A61K 31/7076, A61P 11/00, A61P 39/00

(54) **NOVEL COMPOUND AS ENDOTHELIAL TO MESENCHYMAL TRANSITION (ENDMT) INHIBITOR, AND PHARMACEUTICAL COMPOSITION COMPRISING SAME**

(30) Priority: 21.03.2023 KR 20230036742
(71) Applicant: Korea Institute of Radiological & Medical Sciences, Seoul 01812 (KR); UIF (University Industry Foundation), Yonsei University, Seoul 03722 (KR)
(72) Inventor: LEE, Bong Yong, Seoul 06625 (KR); KIM, Shin Ae, Suwon-si, Gyeonggi-do 16507 (KR); CHO, Young Kyoung, Suwon-si, Gyeonggi-do 16508 (KR); LEE, Yoon Jin, Seoul 07997 (KR); NAM, Jae Kyung, Seoul 01658 (KR); KIM, Ji Hee, Seoul 03763 (KR); CHO, Jae Ho, Seoul 03698 (KR)
(74) Representative: Petty, Catrin Helen
(86) International application number: PCT/KR2024/095623
(87) International publication number: WO 2024/196236

(57) **Abstract**

The present invention relates to a novel compound as an EndMT inhibitor and a pharmaceutical composition comprising the same, and, more specifically, to a novel compound as an EndMT inhibitor and a pharmaceutical composition for treating pulmonary diseases, comprising the same as an active ingredient. The compound according to the present invention exhibits excellent EndMT inhibitory activity, and thus can be effectively used for preventing and treating various pulmonary diseases associated with EndMT activity.

## Description

### TECHNICAL FIELD

The present invention relates to a novel compound as an EndMT inhibitor and a pharmaceutical composition including the same, and more specifically to a novel compound as an EndMT inhibitor and a pharmaceutical composition for treating a pulmonary disease including the same as an active ingredient.

### BACKGROUND ART

Pulmonary fibrosis (PF) is a condition in which lung tissue thickens, hardens, and forms scar tissue. The most common type of pulmonary fibrosis is idiopathic pulmonary fibrosis, which has no specific cause, and it is known that pulmonary fibrosis can be caused by autoimmune diseases such as rheumatoid arthritis, viral infections and gastroesophageal reflux disease, environmental factors, or genetic predisposition, as well as radiation therapy for lung cancer or breast cancer.

Specifically, approximately 60% of non-small cell lung cancer patients receive radiation therapy, but unfortunately, conventional radiation therapy or external beam radiotherapy is known to induce pulmonary disease complications such as pneumonia and pulmonary fibrosis in cancer survivors. Radiation-induced pulmonary fibrosis (RIPF) is a dysfunction of the body that occurs 6 to 12 months after radiation therapy and is characterized by excessive extracellular matrix (ECM) deposition and fibroblast proliferation. Radiation-induced pulmonary fibrosis occurs within weeks after radiation exposure, resulting in narrowing and disappearance of capillaries by endothelial cell (EC) swelling, the growth of new fibrin plugs, and endothelial proliferation, especially in larger vessels such as arteries and veins, with functional microvascular depletion inevitably leading to tissue ischemia and hypoxia.

Meanwhile, activated myofibroblasts play a central role in the production of collagen and ECM proteins during pulmonary fibrosis. Myofibroblasts originate from various cell types, including resident stromal fibroblasts, bone marrow-derived fibroblasts, and epithelial cells undergoing mesenchymal transition (EMT) (Saito A. J Biochem. 2013; 153:493-5). Several studies have shown that alveolar type II epithelial cells undergo epithelial-to-mesenchymal transition during the development of pulmonary fibrosis, including RIPF (Balli D. et al. Foxm1 transcription factor is required for lung fibrosis and epithelial-to-mesenchymal transition. EMBO J. 2013; 32:231-44.; Almeida C. et al. The role of alveolar epithelium in radiation-induced lung injury. PLoS ONE 2013; 8:e53628). In addition, endothelial-to-mesenchymal transition (EndMT) has recently been reported to generate fibroblasts in cardiac and renal fibrosis and cancer (Saito A. J Biochem. 2013; 153:493-5; Potenta S et al. Br J Cancer. 2008; 99:1375-9; van Meeteren LA et al. Cell Tissue Res. 2012; 347:177-86). In addition, EndMT has been demonstrated to serve as a source of fibroblasts in bleomycin-induced pulmonary fibrosis (Hashimoto N. et al. Am J Respir Cell Mol Biol. 2010; 43:161-72.).

EndMT is characterized by the loss of cell-cell junctions and the acquisition of an invasive and migratory phenotype. Mesenchymal cell markers, such as α-smooth muscle actin (α-SMA), fibroblast-specific protein-1 (FSP-1) and vimentin, are upregulated, whereas EC-specific markers including CD31 and vascular endothelial (VE)-cadherin are downregulated.

Treatment options for pulmonary fibrosis are very limited. Some types of pulmonary fibrosis may respond to corticosteroids or other immunosuppressants, but these treatments do not always have positive results and are often ineffective in patients with idiopathic pulmonary fibrosis. In addition, pulmonary fibrosis often follows other pulmonary diseases, and drug interactions with other drugs used in prior therapy can be problematic, thereby causing serious side effects.

In particular, there is currently no drug that can improve radiation-induced pulmonary fibrosis, and although Nintedanib and Pirfenidone, which are currently used as treatments for idiopathic pulmonary fibrosis, have been reported to be effective for radiation-induced pulmonary fibrosis, they do not improve pulmonary fibrosis and only show the effect of slowing the progression of the disease. Glucocorticosteroids, such as prednisone for alleviating inflammation, and antibiotics such as colchicine and penicillamine for inhibiting collagen synthesis, are also used as treatments for radiation-induced pulmonary fibrosis, but these drugs have the problem of being too toxic and causing side effects. In addition, Amifostine, which is a free radical scavenger developed for radiation damage to normal tissues, has high toxicity and is expensive, which limits its commercialization. In addition, the steroid-based antiinflammatory agents that are most commonly used to alleviate pneumonia symptoms caused by radiation cannot be considered fundamental treatments for radiation-induced pulmonary fibrosis.

Accordingly, in the present invention, based on the research results (Choi S. et al., Clin Cancer Res. 2015; 21(16):3716-3726) that EndMT is a major pathogenesis in radiation-induced diseases, the inventors of the present invention have made diligent efforts to develop a novel compound that can treat related diseases, such as radiation-induced pneumonia or pulmonary fibrosis, by inhibiting EndMT, and as a result, the present invention was completed by newly synthesizing a novel compound that can effectively inhibit EndMT.

### DISCLOSURE

### TECHNICAL PROBLEM

An object of the present invention is to provide a novel compound that is capable of inhibiting EndMT and a pharmaceutical use thereof.

### TECHNICAL SOLUTION

In order to achieve the above object, the present invention provides a compound represented by Chemical Formula (1) below, an optical isomer thereof, a racemic mixture thereof, a hydrate thereof, a solvate thereof or a pharmaceutically acceptable salt thereof:

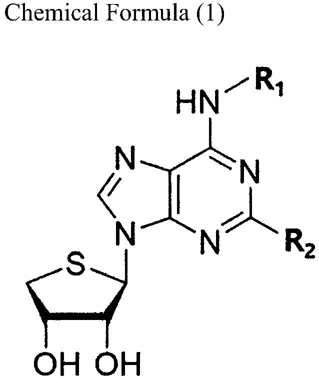

Here, R₁ is a fused bicyclic ring in which an aryl ring and a non-aromatic cycloalkyl ring are fused, wherein the fused bicyclic ring is substituted or unsubstituted and
R₂ is hydrogen (H), halogen (X) or an alkynyl group.

In the present invention, the two-ring fused ring may be benzocycloalkyl.

In the present invention, the substituted two-ring fused ring may be constituted such that any one or more hydrogens of an aryl ring and a non-aromatic cycloalkyl ring are independently substituted with C₁₋₆ alkoxy, halogen, C₁₋₆ alkyl, CF₃, CN, CO₂Me, CONH₂ or COOH.

In the present invention, the alkynyl group may be a ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group or a 1,1-dimethyl-2-butynyl group.

In the present invention, the compound may be selected from the following group:
(1) (2R,3R,4S)-2-[6-[[(1R)-indan-1-yl]amino]-2-prop-1-ynyl-purin-9-yl]tetrahydrothiophene-3,4-diol;
(2) (2R,3R,4S)-2-[6-[[(1S)-indan-1-yl]amino]-2-prop-1-ynyl-purin-9-yl]tetrahydrothiophene-3,4-diol;
(3) (2R,3R,4S)-2-[2-but-1-ynyl-6-[[(1R)-indan-1-yl]amino]purin-9-yl]tetrahydrothiophene-3,4-diol;
(4) (2R,3R,4S)-2-[2-but-1-ynyl-6-[[(1S)-indan-1-yl]amino]purin-9-yl]tetrahydrothiophene-3,4-diol;
(5) (2R,3R,4S)-2-[6-[[(1R)-indan-1-yl]amino]-2-pent-1-ynyl-purin-9-yl]tetrahydrothiophene-3,4-diol;
(6) (2R,3R,4S)-2-[6-[[(1S)-indan-1-yl]amino]-2-pent-1-ynyl-purin-9-yl]tetrahydrothiophene-3,4-diol;
(7) (2R,3R,4S)-2-[2-chloro-6-(indan-2-ylamino)purin-9-yl]tetrahydrothiophene-3,4-diol;
(8) (2R,3R,4S)-2-[2-chloro-6-(indan-1-ylamino)purin-9-yl]tetrahydrothiophene-3,4-diol;
(9) (2R,3R,4S)-2-[2-chloro-6-[[(1R)-indan-1-yl]amino]purin-9-yl]tetrahydrothiophene-3,4-diol;
(10) (2R,3R,4S)-2-[2-chloro-6-[[(1S)-indan-1-yl]amino]purin-9-yl]tetrahydrothiophene-3,4-diol;
(11) (2R,3R,4S)-2-[2-chloro-6-[[(1R)-4-methoxyindan-1-yl]amino]purin-9-yl]tetrahydrothiophene-3,4-diol;
(12) (2R,3R,4S)-2-[2-chloro-6-[[(1R)-5-methoxyindan-1-yl]amino]purin-9-yl]tetrahydrothiophene-3,4-diol;
(13) (2R,3R,4S)-2-[2-chloro-6-[[(1R)-6-methoxyindan-1-yl]amino]purin-9-yl]tetrahydrothiophene-3,4-diol;
(14) (2R,3R,4S)-2-[2-chloro-6-[[(1R)-7-methoxyindan-1-yl]amino]purin-9-yl]tetrahydrothiophene-3,4-diol;
(15) (2R,3R,4S)-2-[2-chloro-6-[[(1R)-4-fluoroindan-1-yl]amino]purin-9-yl]tetrahydrothiophene-3,4-diol;
(16) (2R,3R,4S)-2-[2-chloro-6-[[(1R)-5-fluoroindan-1-yl]amino]purin-9-yl]tetrahydrothiophene-3,4-diol;
(17) (2R,3R,4S)-2-[2-chloro-6-[[(1R)-4-chloroindan-1-yl]amino]purin-9-yl]tetrahydrothiophene-3,4-diol;
(18) (2R,3R,4S)-2-[2-chloro-6-[[(1R)-5-chloroindan-1-yl]amino]purin-9-yl]tetrahydrothiophene-3,4-diol;
(19) (2R,3R,4S)-2-[2-chloro-6-[[(1R)-4-bromoindan-1-yl]amino]purin-9-yl]tetrahydrothiophene-3,4-diol;
(20) (2R,3R,4S)-2-[2-chloro-6-[[(1R)-5-bromoindan-1-yl]amino]purin-9-yl]tetrahydrothiophene-3,4-diol;
(21) (2R,3R,4S)-2-[2-chloro-6-[[(1R)-6-bromoindan-1-yl]amino]purin-9-yl]tetrahydrothiophene-3,4-diol;
(22) (2R,3R,4S)-2-[2-chloro-6-[[(1R)-4-methylindan-1-yl]amino]purin-9-yl]tetrahydrothiophene-3,4-diol;
(23) (2R,3R,4S)-2-[2-chloro-6-[[(1R)-4-(trifluoromethyl)indan-1-yl]amino]purin-9-yl]tetrahydrothiophene-3,4-diol;
(24) (1R)-1-[[2-chloro-9-[(2R,3R,4S)-3,4-dihydroxytetrahydrothiophen-2-yl]purin-6-yl]amino]indane-4-carbonitrile;
(25) (R)-1-((2-chloro-9-((2R,3R,4S)-3,4-dihydroxytetrahydrothiophen-2-yl)-9H-purin-6-yl)amino)-2,3-dihydro-1H-indene-4-carboxamide;
(26) (2R,3R,4S)-2-[2-chloro-6-[[(1R)-7-bromoindan-1-yl]amino]purin-9-yl]tetrahydrothiophene-3,4-diol;
(27) (2R,3R,4S)-2-[2-chloro-6-[[(1R)-5-methylindan-1-yl]amino]purin-9-yl]tetrahydrothiophene-3,4-diol;
(28) (2R,3R,4S)-2-[2-chloro-6-[[(1R)-5-(trifluoromethyl)indan-1-yl]amino]purin-9-yl]tetrahydrothiophene-3,4-diol);
(29) (1R)-1-[[2-chloro-9-[(2R,3R,4S)-3,4-dihydroxytetrahydrothiophen-2-yl]purin-6-yl]amino]indane-5-carbonitrile;
(30) Methyl (R)-1-((2-chloro-9-((2R,3R,4S)-3,4-dihydroxytetrahydrothiophen-2-yl)-9H-purin-6-yl)amino)-2,3-dihydro-1H-indene-4-carboxylate;
(31) Methyl (R)-1-((2-chloro-9-((2R,3R,4S)-3,4-dihydroxytetrahydrothiophen-2-yl)-9H-purin-6-yl)amino)-2,3-dihydro-1H-indene-5-carboxylate;
(32) (R)-1-((2-chloro-9-((2R,3R,4S)-3,4-dihydroxytetrahydrothiophen-2-yl)-9H-purin-6-yl)amino)-2,3-dihydro-1H-indene-4-carboxylic acid;
(33) (R)-1-((2-chloro-9-((2R,3R,4S)-3,4-dihydroxytetrahydrothiophen-2-yl)-9H-purin-6-yl)amino)-2,3-dihydro-1H-indene-5-carboxylic acid;
(34) (R)-1-((2-chloro-9-((2R,3R,4S)-3,4-dihydroxytetrahydrothiophen-2-yl)-9H-purin-6-yl)amino)-2,3-dihydro-1H-indene-5-carboxamide;
(35) (2R,3R,4S)-2-(2-chloro-6-((1,2,3,4-tetrahydronaphthalen-2-yl)amino)-9H-purin-9-yl)tetrahydrothiophene-3,4-diol;
(36) (2R,3R,4S)-2-(2-chloro-6-(((S)-1,2,3,4-tetrahydronaphthalen-2-yl)amino)-9H-purin-9-yl)tetrahydrothiophene-3,4-diol;
(37) (2R,3R,4S)-2-(2-chloro-6-(((R)-1,2,3,4-tetrahydronaphthalen-2-yl)amino)-9H-purin-9-yl)tetrahydrothiophene-3,4-diol;
(38) (2R,3R,4S)-2-(2-chloro-6-((1,2,3,4-tetrahydronaphthalen-1-yl)amino)-9H-purin-9-yl)tetrahydrothiophene-3,4-diol;
(39) (2R,3R,4S)-2-(2-chloro-6-(((R)-1,2,3,4-tetrahydronaphthalen-1-yl)amino)-9H-purin-9-yl)tetrahydrothiophene-3,4-diol;
(40) (2R,3R,4S)-2-(2-chloro-6-(((S)-1,2,3,4-tetrahydronaphthalen-1-yl)amino)-9H-purin-9-yl)tetrahydrothiophene-3,4-diol);
(41) (2R,3R,4S)-2-(6-(((R)-bicyclo[4.2.0]octa-1(6),2,4-trien-7-yl)amino)-2-chloro-9H-purin-9-yl)tetrahydrothiophene-3,4-diol;
(42) (2R,3R,4S)-2-(2-chloro-6-((6,7,8,9-tetrahydro-5H-benzo[7]annulen-5-yl)amino)-9H-purin-9-yl)tetrahydrothiophene-3,4-diol;
(43) (2R,3R,4S)-2-(2-chloro-6-((6,7,8,9-tetrahydro-5H-benzo[7]annulen-7-yl)amino)-9H-purin-9-yl)tetrahydrothiophene-3,4-diol;
(44) (2R,3R,4S)-2-(6-(((R)-2,3-dihydro-1H-inden-1-yl)amino)-2-iodo-9H-purin-9-yl)tetrahydrothiophene-3,4-diol; and
(45) (2R,3R,4S)-2-(6-(((R)-2,3-dihydro-1H-inden-1-yl)amino)-9H-purin-9-yl)tetrahydrothiophene-3,4-diol.

In the present invention, the compound, optical isomer thereof, racemic mixture thereof, hydrate thereof, solvate thereof or pharmaceutically acceptable salt thereof may inhibit endothelial-to-mesenchymal transition (EndMT).

In addition, an object of the present invention is to provide a pharmaceutical composition for preventing or treating a pulmonary disease, including the compound, optical isomer thereof, racemic mixture thereof, hydrate thereof, solvate thereof or pharmaceutically acceptable salt thereof as an active ingredient.

In the present invention, the pulmonary disease may be pneumonia, pulmonary fibrosis or lung cancer.

In the present invention, the pulmonary disease may be a radiation-induced pulmonary disease.

In the present invention, the pharmaceutical composition may be used in conjunction with radiation therapy.

In the present invention, the pharmaceutical composition may be administered before and/or after radiation therapy.

In addition, an object of the present invention is to provide n endothelial-to-mesenchymal transition (EndMT) inhibitor, including the compound, optical isomer thereof, racemic mixture thereof, hydrate thereof, solvate thereof or salt thereof as an active ingredient.

In the present invention, the compound, optical isomer thereof, racemic mixture thereof, hydrate thereof, solvate thereof or salt thereof may inhibit actin stress fiber formation in endothelial cells.

### ADVANTAGEOUS EFFECTS

The compound according to the present invention exhibits excellent EndMT inhibitory activity, and thus has the advantage of being effectively used for the prevention and treatment of various pulmonary diseases associated with EndMT activity.

### MODES OF THE INVENTION

The descriptions disclosed in the present specification or application are merely illustrative for the purpose of described embodiments according to the technical idea of the present invention, and embodiments according to the technical idea of the present invention can be implemented in various forms other than the embodiments disclosed in the present specification or application, and the technical idea of the present invention is not construed as being limited to the embodiments described in the present specification or application.

Hereinafter, the present invention will be described in detail.

In the present invention, a novel compound having EndMT inhibitory activity was developed.

Accordingly, in one aspect, the present invention relates to a compound represented by Chemical Formula (1), an optical isomer thereof, a racemic mixture thereof, a hydrate thereof, a solvate thereof or a pharmaceutically acceptable salt thereof:

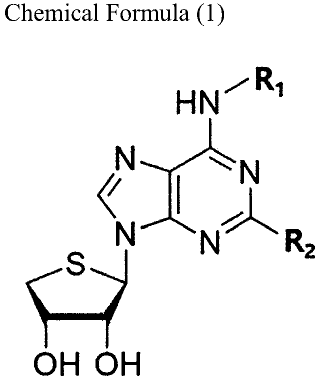

In Chemical Formula (1) above, R₁ is a fused bicyclic ring in which an aryl ring and a non-aromatic cycloalkyl ring are fused, wherein the fused bicyclic ring is substituted or unsubstituted and
R₂ is hydrogen (H), halogen (X) or an alkynyl group.

In the present invention, the fused bicyclic ring may be benzocycloalkyl, but is not limited thereto.

In the present invention, the fused bicyclic ring may be a fused bicyclic ring in which a C₄₋₈ cycloalkyl ring is fused to a benzene ring (phenyl), but is not limited thereto.

In one embodiment, the fused bicyclic ring may be constituted such that any one or more hydrogens of an aryl ring and a non-aromatic cycloalkyl ring are independently substituted with OCH₃, F, Cl, Br, I, CH₃, CF₃, CN, CO₂Me, CONH₂ or COOH.

In one embodiment, the fused bicyclic ring may be a fused bicyclic ring in which a benzene ring and a cyclobutyl ring are fused, as in

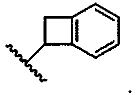

In another embodiment, the fused bicyclic ring may be a fused bicyclic ring in which a benzene ring and a cyclopentyl ring are fused, as in 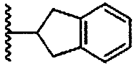 or

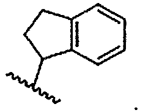

In still another embodiment, the fused bicyclic ring may be a fused bicyclic ring in which a benzene ring and a cyclohexyl ring are fused, as in 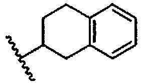 or

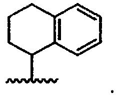

In still another embodiment, the fused bicyclic ring may be a fused bicyclic ring in which a benzene ring and a cycloheptyl ring are fused, as in

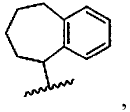

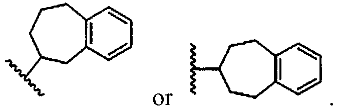

In the present invention, the substituted fused bicyclic ring may be characterized in that any one or more hydrogen atoms in the aryl ring and the non-aromatic cycloalkyl ring are independently substituted with C₁₋₆ alkoxy, halogen, C₁₋₆ alkyl, CF₃, CN, CO₂Me, CONH₂ or COOH, but is not limited thereto.

In one embodiment, the substituted fused bicyclic ring may be characterized in that any one or more hydrogens in any fused bicyclic ring selected from the group consisting of the following structural formulae are independently substituted with OCH₃, F, Cl, Br, I, CH₃, CF₃, CN, CO₂Me, CONH₂ or COOH, but is not limited thereto:

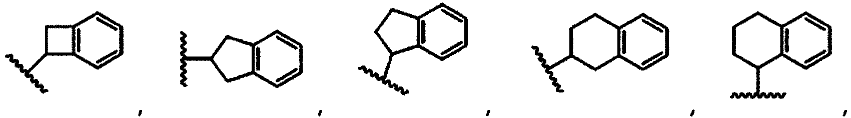

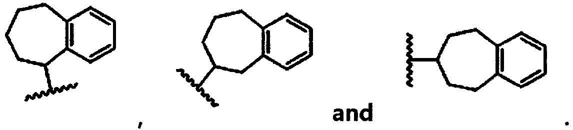

Specifically, in the substituted fused bicyclic ring, R₁ may be any one selected from the group consisting of the following structural formulae:

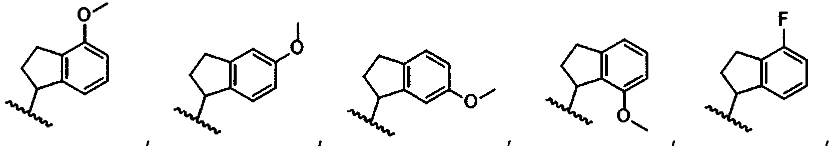

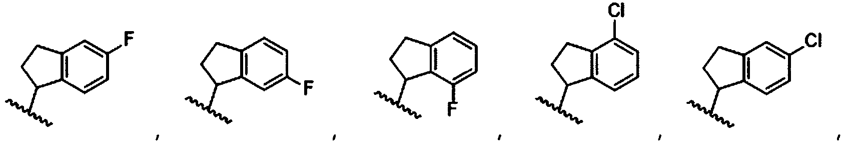

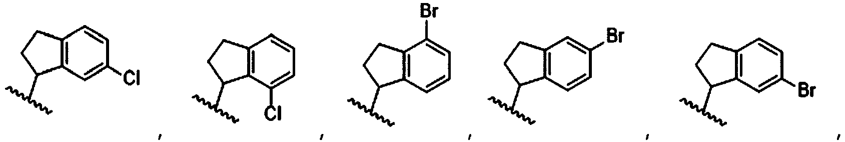

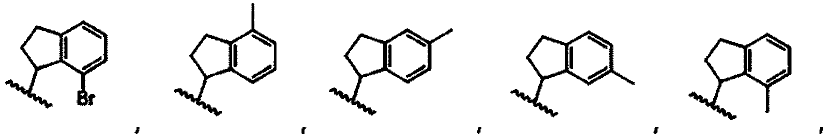

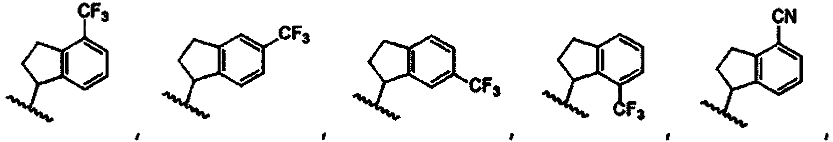

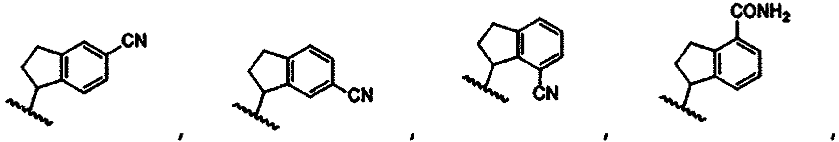

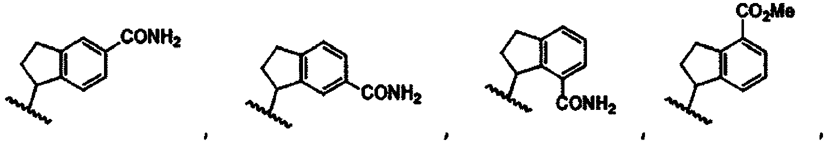

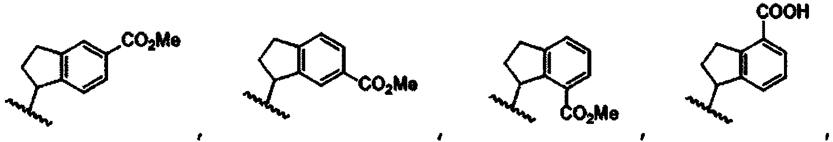

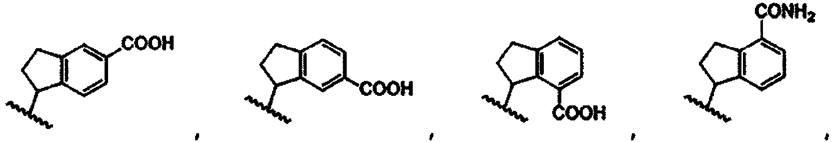

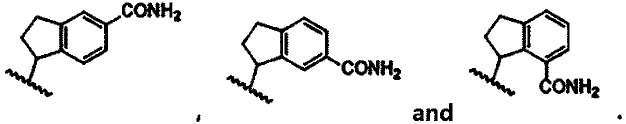

In one embodiment, the alkynyl group may be an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group or a 1,1-dimethyl-2-butynyl group but is not limited to.

In one embodiment, R₂ may be H, F, Cl, Br, I, 1-propynyl, 2-propynyl, 1-butynyl, 2-butynyl, 1-pentynyl or 2-pentynyl.

In one embodiment, R₂ may be

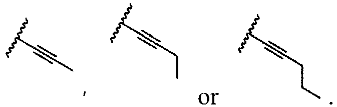

In the present invention, the compound may be any one of Examples 1 to 45 below, and it may be selected from the following group.
(1) (2R,3R,4S)-2-[6-[[(1R)-indan-1-yl]amino]-2-prop-1-ynyl-purin-9-yl]tetrahydrothiophene-3,4-diol;
(2) (2R,3R,4S)-2-[6-[[(1S)-indan-1-yl]amino]-2-prop-1-ynyl-purin-9-yl]tetrahydrothiophene-3,4-diol;
(3) (2R,3R,4S)-2-[2-but-1-ynyl-6-[[(1R)-indan-1-yl]amino]purin-9-yl]tetrahydrothiophene-3,4-diol;
(4) (2R,3R,4S)-2-[2-but-1-ynyl-6-[[(1S)-indan-1-yl]amino]purin-9-yl]tetrahydrothiophene-3,4-diol;
(5) (2R,3R,4S)-2-[6-[[(1R)-indan-1-yl]amino]-2-pent-1-ynyl-purin-9-yl]tetrahydrothiophene-3,4-diol;
(6) (2R,3R,4S)-2-[6-[[(1S)-indan-1-yl]amino]-2-pent-1-ynyl-purin-9-yl]tetrahydrothiophene-3,4-diol;
(7) (2R,3R,4S)-2-[2-chloro-6-(indan-2-ylamino)purin-9-yl]tetrahydrothiophene-3,4-diol;
(8) (2R,3R,4S)-2-[2-chloro-6-(indan-1-ylamino)purin-9-yl]tetrahydrothiophene-3,4-diol;
(9) (2R,3R,4S)-2-[2-chloro-6-[[(1R)-indan-1-yl]amino]purin-9-yl]tetrahydrothiophene-3,4-diol;
(10) (2R,3R,4S)-2-[2-chloro-6-[[(1S)-indan-1-yl]amino]purin-9-yl]tetrahydrothiophene-3,4-diol;
(11) (2R,3R,4S)-2-[2-chloro-6-[[(1R)-4-methoxyindan-1-yl]amino]purin-9-yl]tetrahydrothiophene-3,4-diol;
(12) (2R,3R,4S)-2-[2-chloro-6-[[(1R)-5-methoxyindan-1-yl]amino]purin-9-yl]tetrahydrothiophene-3,4-diol;
(13) (2R,3R,4S)-2-[2-chloro-6-[[(1R)-6-methoxyindan-1-yl]amino]purin-9-yl]tetrahydrothiophene-3,4-diol;
(14) (2R,3R,4S)-2-[2-chloro-6-[[(1R)-7-methoxyindan-1-yl]amino]purin-9-yl]tetrahydrothiophene-3,4-diol;
(15) (2R,3R,4S)-2-[2-chloro-6-[[(1R)-4-fluoroindan-1-yl]amino]purin-9-yl]tetrahydrothiophene-3,4-diol;
(16) (2R,3R,4S)-2-[2-chloro-6-[[(1R)-5-fluoroindan-1-yl]amino]purin-9-yl]tetrahydrothiophene-3,4-diol;
(17) (2R,3R,4S)-2-[2-chloro-6-[[(1R)-4-chloroindan-1-yl]amino]purin-9-yl]tetrahydrothiophene-3,4-diol;
(18) (2R,3R,4S)-2-[2-chloro-6-[[(1R)-5-chloroindan-1-yl]amino]purin-9-yl]tetrahydrothiophene-3,4-diol;
(19) (2R,3R,4S)-2-[2-chloro-6-[[(1R)-4-bromoindan-1-yl]amino]purin-9-yl]tetrahydrothiophene-3,4-diol;
(20) (2R,3R,4S)-2-[2-chloro-6-[[(1R)-5-bromoindan-1-yl]amino]purin-9-yl]tetrahydrothiophene-3,4-diol;
(21) (2R,3R,4S)-2-[2-chloro-6-[[(1R)-6-bromoindan-1-yl]amino]purin-9-yl]tetrahydrothiophene-3,4-diol;
(22) (2R,3R,4S)-2-[2-chloro-6-[[(1R)-4-methylindan-1-yl]amino]purin-9-yl]tetrahydrothiophene-3,4-diol;
(23) (2R,3R,4S)-2-[2-chloro-6-[[(1R)-4-(trifluoromethyl)indan-1-yl]amino]purin-9-yl]tetrahydrothiophene-3,4-diol;
(24) (1R)-1-[[2-chloro-9-[(2R,3R,4S)-3,4-dihydroxytetrahydrothiophen-2-yl]purin-6-yl]amino]indane-4-carbonitrile;
(25) (R)-1-((2-chloro-9-((2R,3R,4S)-3,4-dihydroxytetrahydrothiophen-2-yl)-9H-purin-6-yl)amino)-2,3-dihydro-1H-indene-4-carboxamide;
(26) (2R,3R,4S)-2-[2-chloro-6-[[(1R)-7-bromoindan-1-yl]amino]purin-9-yl]tetrahydrothiophene-3,4-diol;
(27) (2R,3R,4S)-2-[2-chloro-6-[[(1R)-5-methylindan-1-yl]amino]purin-9-yl]tetrahydrothiophene-3,4-diol;
(28) (2R,3R,4S)-2-[2-chloro-6-[[(1R)-5-(trifluoromethyl)indan-1-yl]amino]purin-9-yl]tetrahydrothiophene-3,4-diol);
(29) (1R)-1-[[2-chloro-9-[(2R,3R,4S)-3,4-dihydroxytetrahydrothiophen-2-yl]purin-6-yl]amino]indane-5-carbonitrile;
(30) Methyl (R)-1-((2-chloro-9-((2R,3R,4S)-3,4-dihydroxytetrahydrothiophen-2-yl)-9H-purin-6-yl)amino)-2,3-dihydro-1H-indene-4-carboxylate;
(31) Methyl (R)-1-((2-chloro-9-((2R,3R,4S)-3,4-dihydroxytetrahydrothiophen-2-yl)-9H-purin-6-yl)amino)-2,3-dihydro-1H-indene-5-carboxylate;
(32) (R)-1-((2-chloro-9-((2R,3R,4S)-3,4-dihydroxytetrahydrothiophen-2-yl)-9H-purin-6-yl)amino)-2,3-dihydro-1H-indene-4-carboxylic acid;
(33) (R)-1-((2-chloro-9-((2R,3R,4S)-3,4-dihydroxytetrahydrothiophen-2-yl)-9H-purin-6-yl)amino)-2,3-dihydro-1H-indene-5-carboxylic acid;
(34) (R)-1-((2-chloro-9-((2R,3R,4S)-3,4-dihydroxytetrahydrothiophen-2-yl)-9H-purin-6-yl)amino)-2,3-dihydro-1H-indene-5-carboxamide;
(35) (2R,3R,4S)-2-(2-chloro-6-((1,2,3,4-tetrahydronaphthalen-2-yl)amino)-9H-purin-9-yl)tetrahydrothiophene-3,4-diol;
(36) (2R,3R,4S)-2-(2-chloro-6-(((S)-1,2,3,4-tetrahydronaphthalen-2-yl)amino)-9H-purin-9-yl)tetrahydrothiophene-3,4-diol;
(37) (2R,3R,4S)-2-(2-chloro-6-(((R)-1,2,3,4-tetrahydronaphthalen-2-yl)amino)-9H-purin-9-yl)tetrahydrothiophene-3,4-diol;
(38) (2R,3R,4S)-2-(2-chloro-6-((1,2,3,4-tetrahydronaphthalen-1-yl)amino)-9H-purin-9-yl)tetrahydrothiophene-3,4-diol;
(39) (2R,3R,4S)-2-(2-chloro-6-(((R)-1,2,3,4-tetrahydronaphthalen-1-yl)amino)-9H-purin-9-yl)tetrahydrothiophene-3,4-diol;
(40) (2R,3R,4S)-2-(2-chloro-6-(((S)-1,2,3,4-tetrahydronaphthalen-1-yl)amino)-9H-purin-9-yl)tetrahydrothiophene-3,4-diol);
(41) (2R,3R,4S)-2-(6-(((R)-bicyclo[4.2.0]octa-1(6),2,4-trien-7-yl)amino)-2-chloro-9H-purin-9-yl)tetrahydrothiophene-3,4-diol;
(42) (2R,3R,4S)-2-(2-chloro-6-((6,7,8,9-tetrahydro-5H-benzo[7]annulen-5-yl)amino)-9H-purin-9-yl)tetrahydrothiophene-3,4-diol;
(43) (2R,3R,4S)-2-(2-chloro-6-((6,7,8,9-tetrahydro-5H-benzo[7]annulen-7-yl)amino)-9H-purin-9-yl)tetrahydrothiophene-3,4-diol;
(44) (2R,3R,4S)-2-(6-(((R)-2,3-dihydro-1H-inden-1-yl)amino)-2-iodo-9H-purin-9-yl)tetrahydrothiophene-3,4-diol; and
(45) (2R,3R,4S)-2-(6-(((R)-2,3-dihydro-1H-inden-1-yl)amino)-9H-purin-9-yl)tetrahydrothiophene-3,4-diol.

In the present invention, the compound, optical isomer thereof, racemic mixture thereof, hydrate thereof, solvate thereof or pharmaceutically acceptable salt thereof may be characterized by inhibiting endothelial-to-mesenchymal transition (EndMT).

In the present invention, when defining the compound of Chemical Formula (1), the following definitions apply unless otherwise stated.

The term "alkyl" refers to a straight-chain or branched-chain saturated hydrocarbon, and preferably C₁₋₁₀ alkyl. For example, the alkyl includes methyl, ethyl, n-propyl, iso-propyl, n-butyl, iso-butyl, tert-butyl, n-pentyl, iso-pentyl, n-hexyl, 3-methylhexyl, 2,2-dimethylpentyl, 2,3-dimethylpentyl, n-heptyl, n-octyl, n-nonyl and n-decyl, but is not limited thereto.

As used herein, the term "aryl" means an aromatic hydrocarbon, including a polycyclic aromatic ring system in which a carbocyclic aromatic ring or a heteroaryl ring is fused with one or more other rings. Preferably, it is C₅₋₁₂ aryl, and more preferably C₅₋₁₀ aryl. For example, the aryl includes phenyl, naphthyl, tetrahydronaphthyl and the like, but is not limited thereto.

The term "cycloalkyl" refers to a partially or fully saturated single or fused ring hydrocarbon, and preferably C₃₋₁₀ cycloalkyl. For example, it includes cyclopropyl, cyclobutyl, cyclopentyl, cyclohexyl, cyclohexynyl and the like, but is not limited thereto.

The term "halogen" or "halo" refers to fluorine/fluorine (F), chlorine (Cl), bromine (Br) or iodine (I).

The term "alkynyl group" refers to an unsaturated branched or straight hydrocarbon group having two or more carbon atoms and at least one triple bond. In the present invention, the alkynyl group may include, but is not limited to, an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, a 1,1-dimethyl-2-butynyl group and the like.

Meanwhile, phalloidin is a highly selective dicyclic peptide used for staining actin filaments (also called F-actin), and the filaments in the EndMT process are characterized by forming actin stress fibers (Maddaluno L. et al., EndMT contributes to the onset and progression of cerebral cavernous malformations. Nature 2013; 498:492-6.; Wojciech Micha 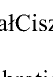 Ciszewski et al., Cytoskeleton Reorganization in EndMT - The Role in Cancer and Fibrotic Diseases. *Int J Mol Sci.* 2021 Nov; 22(21): 11607). In the present invention, by confirming the effect of inhibiting phalloidin by a novel compound, the inhibitory effect of actin stress fiber formation, that us, the EndMT inhibitory effect, was confirmed.

The EndMT mechanism is one of the mechanisms that induce fibrosis, and it is known that radiation induces EndMT of pulmonary vascular endothelial cells and causes the transformation of mesenchymal stem cells, which then secrete various factors (e.g., TGF-βs) to induce the proliferation of surrounding cells (smooth muscle cells, epithelial cells) and transformation into myofibroblasts, thereby causing pulmonary fibrosis. It has been reported that when EndMT is reduced in a radiation-induced pulmonary fibrosis model, pulmonary fibrosis is reduced (Seo-Hyun Choi et al., A Hypoxia-Induced Vascular Endothelial-to-Mesenchymal Transition in Development of Radiation-Induced Pulmonary Fibrosis. Clin Cancer Res. 2015 Aug; 21(16): 3716). In addition, EndMT plays a role in the development of idiopathic pulmonary fibrosis by inducing vascular remodeling, and it has been reported that when EndMT is inhibited, pulmonary fibrosis is reduced in a bleomycin-induced pulmonary fibrosis model. (Hashimoto N et al., 2010. Endothelial mesenchymal transition in bleomycin-induced pulmonary fibrosis. Am J Respir Cell Mol Biol. 43(2):161-172., Eunsik Yun et al., 2023. Ginsenoside Rg3 attenuates pulmonary fibrosis by inhibiting endothelial to mesenchymal transition, Animal Cells and Systems, 27:1, 159-170). Additionally, EndMT has been reported to promote carcinogenesis through the accumulation of cancer-associated fibroblasts (CAF), and the inhibition of EndMT has been reported to increase the therapeutic efficacy of lung cancer. (Choi, K.J. et al. Endothelial-to-mesenchymal transition in anticancer therapy and normal tissue damage. Exp Mol Med 52, 781-792 (2020), Clere N, Renault S and Corre I (2020) Endothelial-to-Mesenchymal Transition in Cancer. Front. Cell Dev. Biol. 8:747.). In this context, the inventors of the present invention have confirmed by using an animal model that the compound according to the present invention can actually exert a therapeutic effect on the disease.

Therefore, the present invention, from another aspect, relates to a compound of Chemical Formula (1), an optical isomer thereof, a racemic mixture thereof, a hydrate thereof, a solvate thereof or a pharmaceutically acceptable salt thereof, for use in inhibiting EndMT and a pharmaceutical composition for preventing or treating a disease associated with EndMT activity.

Specifically, the EndMT inhibition use and disease associated with EndMT activity may be a pulmonary disease.

In one aspect, the present invention relates to a pharmaceutical composition for preventing or treating a pulmonary disease, including the compound, optical isomer thereof, racemic mixture thereof, hydrate thereof, solvate thereof or pharmaceutically acceptable salt thereof as an active ingredient.

In the present invention, the pulmonary disease may be characterized as pneumonia, pulmonary fibrosis or lung cancer, but is not limited thereto.

In the present invention, the pulmonary disease may be characterized as being a radiation-induced pulmonary disease, but is not limited thereto.

In one embodiment, the pulmonary fibrosis may be radiation-induced pulmonary fibrosis or idiopathic pulmonary fibrosis.

In another embodiment, the lung cancer may be small cell lung cancer or non-small cell lung cancer.

When the compound according to the present invention, an optical isomer thereof, a racemic mixture thereof, a hydrate thereof, a solvate thereof or a pharmaceutically acceptable salt thereof is used for the prevention or treatment of a pulmonary disease, it may be characterized in that it is used in conjunction with radiation therapy, but is not limited thereto.

When the compound according to the present invention, an optical isomer thereof, a racemic mixture thereof, a hydrate thereof, a solvate thereof or a pharmaceutically acceptable salt thereof is used in conjunction with radiotherapy, the compound according to the present invention, an optical isomer thereof, a racemic mixture thereof, a hydrate thereof, a solvate thereof or a pharmaceutically acceptable salt thereof may suppress side effects of radiotherapy while enhancing the therapeutic effect of radiotherapy.

When the compound according to the present invention, an optical isomer thereof, a racemic mixture thereof, a hydrate thereof, a solvate thereof or a pharmaceutically acceptable salt thereof is used as an active ingredient for the prevention or treatment of a pulmonary disease, such as pneumonia, fibrosis or lung cancer, the compound according to the present invention, an optical isomer thereof, a racemic mixture thereof, a hydrate thereof, a solvate thereof or a pharmaceutically acceptable salt thereof may be administered before and/or after radiotherapy.

In one embodiment, when the compound according to the present invention, an optical isomer thereof, a racemic mixture thereof, a hydrate thereof, a solvate thereof or a pharmaceutically acceptable salt thereof is used as an active ingredient for the prevention or treatment of a pulmonary disease, such as pneumonia, fibrosis or lung cancer, it may be characterized in that it is administered about 48 hours to about 10 minutes before radiation therapy, and for example, about 36 hours to about 6 hours before radiation therapy or about 24 hours to about 12 hours before radiation therapy, but is not limited thereto.

In another embodiment, when the compound according to the present invention, an optical isomer thereof, a racemic mixture thereof, a hydrate thereof, a solvate thereof or a pharmaceutically acceptable salt thereof is used for the purpose of preventing or treating pulmonary fibrosis, it may be administered immediately after radiation therapy up to the 1^{st} day, 2^{nd} day, 3^{rd} day, 4^{th} day, 5^{th} day, 6^{th} day, 7^{th} day, 8^{th} day, 9^{th} day, 10^{th} day, 11^{th} day, 12^{th} day, 13^{th} day, 14^{th} day, 15^{th} day, 16^{th} day, 17^{th} day, 18^{th} day, 19^{th} day, 20^{th} day, 21^{st} day, 22^{nd} day, 23^{rd} day, 24^{th} day, 25^{th} day, 26^{th} day, 27^{th} day, 28^{th} day, 29^{th} day, 30^{th} day, 31^{st} day, 32^{nd} day, 33^{rd} day, 34^{th} day, 35^{th} day, 36^{th} day, 37^{th} day, 38^{th} day, 39^{th} day, 40^{th} day, 41^{st} day, 42^{nd} day, 43^{rd} day, 44^{th} day, 45^{th} day, 46^{th} day, 47^{th} day, 48^{th} day, 49^{th} day, 50^{th} day, 51^{st} day, 52^{nd} day, 53^{rd} day, 54^{th} day, 55^{th} day, 56^{th} day, 57^{th} day, 58^{th} day, 59^{th} day, 60^{th} day, 61^{st} day, 62^{nd} day, 63^{rd} day, 64^{th} day, 65^{th} day, 66^{th} day, 67^{th} day, 68^{th} day, 69^{th} day, 70^{th} day, 71^{st} day, 72^{nd} day, 73^{rd} day, 74^{th} day, 75^{th} day, 76^{th} day, 77^{th} day, 78^{th} day, 79^{th} day, 80^{th} day, 81^{st} day, 82^{nd} day, 83^{rd} day, 84^{th} day, 85^{th} day, 86^{th} day, 87^{th} day, 88^{th} day, 89^{th} day, 90^{th} day, 91^{st} day, 92^{nd} day, 93^{rd} day, 94^{th} day, 95^{th} day, 96^{th} day, 97^{th} day, 98^{th} day, 99^{th} day or 100^{th} day, but is not limited thereto.

The compound represented by Chemical Formula (1) according to the present invention may be prepared and used in the form of a prodrug, a hydrate, a solvate and a pharmaceutically acceptable salt in order to promote absorption *in vivo* or increase solubility, and therefore, the prodrug, hydrate, solvate and pharmaceutically acceptable salt also fall within the scope of the present invention. In addition, the compound represented by Chemical Formula (1) has a chiral carbon, and thus, the stereoisomers thereof exist, and such stereoisomers are also included within the scope of the present invention.

The term "prodrug" refers to a substance that is transformed *in vivo* into a parent drug. Prodrugs are often used because, in some cases, they are easier to administer than the parent drug. For example, they may be bioactive by oral administration, whereas the parent drug may not be. Prodrugs may also have improved solubility in pharmaceutical compositions than the parent drug. For example, prodrugs may be bio-hydrolyzable esters of compounds according to the invention and pharmaceutically acceptable salts thereof. Another example of a prodrug may be a short peptide (polyamino acid) that is linked to an acid group that is metabolized to reveal the active site of the peptide.

The term "hydrate" means a compound of the present invention or a salt thereof which includes a stoichiometric or non-stoichiometric amount of water bound by non-covalent intermolecular forces.

The term "solvate" means a compound of the present invention or a salt thereof which includes a stoichiometric or non-stoichiometric amount of a solvent bound by non-covalent intermolecular forces. Preferred solvents therefor include those which are volatile, non-toxic and/or suitable for administration to humans.

The term "isomer" means a compound of the present invention or a salt thereof which has the same chemical formula or molecular formula but is structurally or sterically different. Such isomers include structural isomers such as tautomers, and stereoisomers such as R or S isomers having an asymmetric carbon center, and geometric isomers (trans, cis). All of these isomers and mixtures thereof are also included in the scope of the present invention.

The term "pharmaceutically acceptable salt" means a salt form of a compound which does not cause serious irritation to an organism to which the compound is administered and does not impair the biological activity and physical properties of the compound. The pharmaceutical salts include acid addition salts formed by acids which form non-toxic acid addition salts containing pharmaceutically acceptable anions, for example, inorganic acids such as hydrochloric acid, sulfuric acid, nitric acid, phosphoric acid, hydrobromic acid, hydroiodic acid and the like, organic carbonxylic acids such as tartaric acid, formic acid, citric acid, acetic acid, trichloroacetic acid, trifluoroacetic acid, gluconic acid, benzoic acid, lactic acid, fumaric acid, maleic acid, salicylic acid and the like, and sulfonic acids such as methanesulfonic acid, ethanesulfonic acid, benzenesulfonic acid, p-toluenesulfonic acid and the like. For example, pharmaceutically acceptable carboxylic acid salts include metal salts or alkaline earth metal salts formed by lithium, sodium, potassium, calcium, magnesium and the like, amino acid salts such as lysine, arginine, guanidine and the like, organic salts such as dicyclohexylamine, N-methyl-D-glucamine, tris(hydroxymethyl)methylamine, diethanolamine, choline, trimethylamine and the like. The compound of Chemical Formula (1) according to the present invention may be converted into its salt by a conventional method.

In addition, the present invention provides a method for preparing a compound of Chemical Formula (1). The synthetic methods of Examples 1 to 45 are exemplified as a method for preparing a compound of Chemical Formula (1) of the present invention, and the synthetic methods of Examples 1 to 45 do not limit the method for preparing a compound of Chemical Formula (1) according to the present invention. The synthetic methods of Examples 1 to 45 are merely examples, and it is apparent that they can be easily modified by those skilled in the art depending on a specific substituent.

In addition, the present invention provides a pharmaceutical composition for preventing or treating a disease associated with EndMT activity, including the compound of Chemical Formula (1), an optical isomer thereof, a racemic mixture thereof, a hydrate thereof, a solvate thereof or a pharmaceutically acceptable salt thereof as an active ingredient.

In addition, the present invention provides a method for preventing or treating a disease (e.g., pulmonary disease) associated with EndMT inhibition and/or EndMT activity, including administering the compound of Chemical Formula (1), an optical isomer thereof, a racemic mixture thereof, a hydrate thereof, a solvate thereof, or a pharmaceutically acceptable salt thereof to a patient in need thereof.

In addition, the present invention provides a compound of Chemical Formula (1), an optical isomer thereof, a racemic mixture thereof, a hydrate thereof, a solvate thereof or a pharmaceutically acceptable salt thereof for use in inhibiting EndMT and/or for use in preventing or treating a disease associated with EndMT activity (e.g., pulmonary disease).

In addition, the present invention provides the use of a compound of Chemical Formula (1), an optical isomer thereof, a racemic mixture thereof, a hydrate thereof, a solvate thereof or a pharmaceutically acceptable salt thereof, in the manufacture of a medicament for preventing or treating a disease (e.g., pulmonary disease) associated with EndMT inhibition and/or EndMT activity.

In addition, the present invention provides a composition or formulation including the compound of Chemical Formula (1), an optical isomer thereof, a racemic mixture thereof, a hydrate thereof, a solvate thereof or a pharmaceutically acceptable salt thereof, and pharmaceutically acceptable additives.

The composition may be a pharmaceutical composition for treating a disease (e.g., pulmonary disease) associated with EndMT inhibition and/or EndMT activity.

The additives may include pharmaceutically acceptable carriers such as excipients, disintegrants, sweeteners, lubricants or flavoring agents that are commonly used, and may be formulated into oral preparations such as tablets, capsules, powders, granules and suspensions, emulsions or syrups according to conventional methods; or parenteral preparations such as external solutions, external suspensions, external emulsions, gels (ointments, etc.), inhalants, sprays and injections. The preparations may be formulated into various forms, for example, single-dose or multiple-dose dosage forms.

The pharmaceutical composition of the present invention may include excipients such as lactose and corn starch, lubricants such as magnesium stearate, emulsifiers, suspending agents, stabilizers, and isotonic agents. If necessary, a sweetener and/or flavoring agent may be added.

The pharmaceutical composition of the present invention may be administered to mammals such as livestock and humans by various routes, for example, oral, dermal, subcutaneous, intramuscular, intravenous, intraperitoneal, intrarectal, intrauterine, intradural or intracerebrovascular injection and topical administration. Accordingly, the composition of the present invention may be formulated in various forms, such as tablets, capsules, aqueous solutions or suspensions. In the case of tablets for oral administration, carriers such as lactose and corn starch and lubricants such as magnesium stearate may conventionally be added. In the case of capsules for oral administration, lactose and/or dried corn starch may be used as diluents. When an oral aqueous suspension is required, the active ingredient may be combined with an emulsifier and/or a suspending agent. If necessary, a specific sweetener and/or flavoring agent may be added. In the case of intramuscular, intraperitoneal, subcutaneous and intravenous administration, a sterile solution of the active ingredient is usually prepared, and the pH of the solution should be suitably adjusted and buffered. In the case of intravenous administration, the total concentration of the solute should be adjusted so as to impart isotonicity to the preparation. The composition according to the present invention may be in the form of an aqueous solution including a pharmaceutically acceptable carrier, such as saline having a pH of 7.4. The solution may be introduced into the intramuscular bloodstream of the patient by local injection.

The dosage of the active ingredient contained in the pharmaceutical composition of the present invention varies depending on the patient's condition and weight, the degree of the disease, the form of the active ingredient, the route and period of administration, and may be appropriately adjusted depending on the patient. For example, the active ingredient may be administered at a dosage of 0.0001 to 1,000 mg/kg per day, and preferably 0.01 to 100 mg/kg, and the administration may be administered once a day or divided into several times. In addition, the pharmaceutical composition of the present invention may include the active ingredient at a weight percentage of 0.001 to 90% based on the total weight of the composition.

The pharmaceutical composition of the present invention may be administered to mammals such as rats, mice, livestock and humans by various routes, for example, orally, cutaneously, intraperitoneally, rectally, or by intravenous, intramuscular, subcutaneous, intrauterine or intracerebroventricular injection.

The effective amount of the pharmaceutical composition according to the present invention varies depending on the patient's condition and weight, degree of disease, form of active ingredient, route and period of administration, and may be appropriately adjusted depending on the patient. The pharmaceutical composition of the present invention may include the compound, an optical isomer thereof, a racemic mixture thereof, a hydrate thereof, a solvate thereof or a pharmaceutically acceptable salt thereof as an active ingredient in a weight percentage of 0.001 to 90% based on the total weight of the composition.

As used herein, "treating" means, in whole or in part, alleviating a disorder, disease or condition, or one or more of the symptoms associated with the disorder, disease or condition, or slowing or stopping the further progression or worsening of such symptoms, or alleviating or eradicating the cause(s) of the disorder, disease or condition itself.

As used herein, "preventing" means a method of delaying and/or preventing, in whole or in part, the onset, recurrence or spread of a disorder, disease or condition; excluding a subject from acquiring a disorder, disease or condition; or reducing a subject's risk of acquiring a disorder, disease or condition.

The term "effective amount" means an amount that is capable of treating or preventing a disorder, disease or condition, or a symptom thereof, as disclosed herein.

The term "subject" includes an animal, including, but not limited to, a cow, monkey, horse, sheep, pig, chicken, turkey, quail, cat, dog, mouse, rat, rabbit or guinea pig, and in one embodiment, it includes a mammal, and in another embodiment, it includes a human.

In another aspect, the present invention may be provided as an endothelial-to-mesenchymal transition (EndMT) inhibitor including the compound, an optical isomer thereof, a racemic mixture thereof, a hydrate thereof, a solvate thereof or a salt thereof as an active ingredient.

In the present invention, the compound, an optical isomer thereof, a racemic mixture thereof, a hydrate thereof, a solvate thereof or a salt thereof may be characterized by inhibiting actin stress fiber formation in endothelial cells.

The EndMT inhibitor may be an experimental reagent used to inhibit the transition from endothelial cells to mesenchymal cells *in vitro.*

Hereinafter, the present invention will be described in more detail through preparation examples, examples and test example. However, the following preparation examples, examples and test example are intended to illustrate the present invention, and the scope of the present invention is not limited thereto.

### <Preparation Example 1> Preparation of 6-chloro-9-((3aR,4R,6aS)-2,2-dimethyltetrahydrothieno[3,4-d][1,3]dioxol-4-yl)-2-iodo-9H-purine

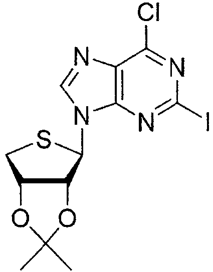

6-Chloro-2-iodopurine (5.01 g, 17.87 mmol) and ACN (89 mL) were added to 250 mL Rbf, N,O-Bis(trimethylsilyl)acetamide (BSA) (6.72 mL, 27.5 mmol) was added under a nitrogen flow, and the mixture was stirred at 40°C for 1 hour. To the reaction mixture, an ACN solution (15 mL) of (3aR,4R,6aS)-2,2-dimethyltetrahydrothieno[3,4-d][1,3]dioxol-4-yl acetate (3.0 g, 13.74 mmol) was slowly added at room temperature, followed by the addition of TMSOTf (2.75 mL). After stirring at 80°C for 3 hours, the reaction mixture was adjusted to pH 8 by adding a saturated NaHCO₃ aqueous solution, and extracted with EtOAc. The organic layer was washed with brine, dried over MgSO₄, filtered and concentrated. The residue obtained by concentration was purified by column chromatography (0-1% MeOH/DCM) to obtain a title compound as an off-white solid (2.88 g, 46%).
¹H NMR (400 MHz, CDCl₃) *δ* 8.06 (s, 1H), 5.85 (s, 1H), 5.33 (t, *J* = 4.7 Hz, 1H), 5.22 (d, *J* = 5.4 Hz, 1H), 3.81 (dd, *J* = 12.8, 4.3 Hz, 1H), 3.26 (d, *J* = 12.8 Hz, 1H), 1.60 (s, 3H), 1.37 (s, 3H); LCMS m/z 439 [M+H]+

### <Preparation Example 2> Preparation of 2,6-dichloro-9-((3aR,4R,6aS)-2,2-dimethyltetrahydrothieno[3,4-d][1,3]dioxol-4-yl)-9H-purine

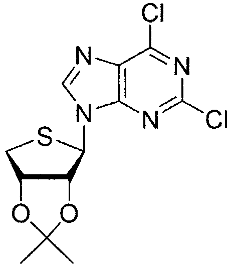

By using the same method as in Preparation Example 1, 2,6-dichloropurine (1.07 g, 5.66 mmol), BSA (2.13 mL, 8.70 mmol), (3aR,4R,6aS)-2,2-dimethyltetrahydrothieno[3,4-d][1,3]dioxol-4-yl acetate (950 mg, 4.35 mmol), ACN (33.2 mL) and TMSOTf (0.87 mL) were used to obtain a title compound as a white solid (1.1 g, 73%).
¹H NMR (400 MHz, CDCl₃) *δ* 8.17 (s, 1H), 5.86 (s, 1H), 5.33 (t, *J* = 4.7 Hz, 1H), 5.21 (d, *J* = 5.3 Hz, 1H), 3.79 (dd, *J* = 12.9, 4.3 Hz, 1H), 3.26 (d, *J* = 12.9 Hz, 1H), 1.59 (s, 3H), 1.37 (s, 3H); LCMS m/z 347 [M+H]+

### <Preparation Example 3> Preparation of (2R,3R,4S)-2-(6-chloro-2-(prop-1-yn-1-yl)-9H-purin-9-yl)tetrahydrothiophene-3,4-diol

### Step 1: Preparation of 6-chloro-9-((3aR,4R,6aS)-2,2-dimethyltetrahydrothieno[3,4-d][1,3]dioxol-4-yl)-2-(prop-1-yn-1-yl)-9H-purine

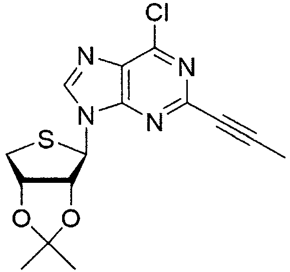

Pd(PPh₃)₄ (203 mg, 0.176 mmol), CuI (37 mg, 0.192 mmol) and CS₂CO₃ (521 mg, 1.6 mmol) were added to a DMF (16 mL) solution of 6-chloro-9-((3aR,4R,6aS)-2,2-dimethyltetrahydrothieno[3,4-d][1,3]dioxol-4-yl)-2-iodo-9H-purine (700 mg, 1.6 mmol), and the mixture was degassed. Propyne (1 M in DMF, 4.8 mL) was added, and the mixture was stirred at room temperature for 3.5 hours. The reaction mixture was concentrated and used in the next reaction without further purification.
LCMS m/z 351 [M+H]+

### Step 2: Preparation of (2R,3R,4S)-2-(6-chloro-2-(prop-1-yn-1-yl)-9H-purin-9-yl)tetrahydrothiophene-3,4-diol

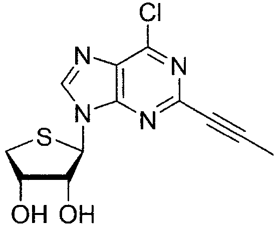

6-Chloro-9-((3aR,4R,6aS)-2,2-dimethyltetrahydrothieno[3,4-d][1,3]dioxol-4-yl)-2-(prop-1-yn-1-yl)-9H-purine (1.6 mmol) was dissolved in 80% HCOOH (40 mL) and stirred at room temperature for 4 hours. EtOAc was added to the reaction mixture, and the mixture was washed with a saturated NaHCO₃ aqueous solution. The organic layer was dried over MgSO₄, filtered and concentrated. The concentrated residue was purified by column chromatography (0-3% MeOH/DCM) to obtain a title compound as a white solid (279 mg, 2 steps 56%).
¹H NMR (400 MHz, CD₃OD) *δ* 8.88 (s, 1H), 6.10 (d, *J* = 6.6 Hz, 1H), 4.72 (dd, *J* = 6.6, 3.4 Hz, 1H), 4.49 (dd, *J* = 7.3, 3.6 Hz, 1H), 3.57 (dd, *J* = 11.0, 4.3 Hz, 1H), 2.98 (dd, *J* = 11.1, 3.5 Hz, 1H), 2.13 (s, 3H); LCMS m/z 311 [M+H]+

### <Preparation Example 4> Preparation of (2R,3R,4S)-2-(2-(but-1-yn-1-yl)-6-chloro-9H-purin-9-yl)tetrahydrothiophene-3,4-diol

### Step 1: Preparation of 2-(but-1-yn-1-yl)-6-chloro-9-((3aR,4R,6aS)-2,2-dimethyltetrahydrothieno[3,4-d][1,3]dioxol-4-yl)-9H-purine

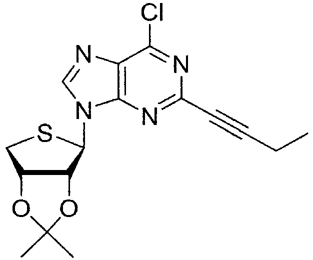

6-Chloro-9-((3aR,4R,6aS)-2,2-dimethyltetrahydrothieno[3,4-d][1,3]dioxol-4-yl)-2-iodo-9H-purine (500 mg, 1.14 mmol) was dissolved in DMF (7.7 mL), and Pd(PPh₃)₄ (133 mg, 0.114 mmol), CuI (26 mg, 0.137 mmol) and CS₂CO₃ (372 mg, 1.14 mmol) were added and degassed. The reaction mixture was stirred for 3 hours while bubbling with 1-butyne (gas). The reaction mixture was diluted with EtOAc, washed with DW and brine, dried over MgSO₄, filtered and concentrated. The concentrated residue was purified by column chromatography (0-25% EtOAc/HX) to obtain a title compound as a yellow solid (295 mg, 71%).
¹H NMR (400 MHz, CDCl₃) *δ* 8.16 (s, 1H), 5.91 (s, 1H), 5.33 (t, *J* = 4.8 Hz, 1H), 5.22 (d, *J* = 5.3 Hz, 1H), 3.79 (dd, *J* = 12.9, 4.3 Hz, 1H), 3.25 (d, *J* = 12.8 Hz, 1H), 2.51 (q, *J* = 7.5 Hz, 2H), 1.59 (s, 3H), 1.37 (s, 3H), 1.30 (t, *J* = 7.5 Hz, 3H); LCMS m/z 365 [M+H]+

### Step 2: Preparation of (2R,3R,4S)-2-(2-(but-1-yn-1-yl)-6-chloro-9H-purin-9-yl)tetrahydrothiophene-3,4-diol

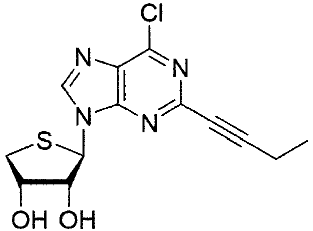

By using the same method as in Step 2 of Preparation Example 3, 2-(but-1-yn-1-yl)-6-chloro-9-((3aR,4R,6aS)-2,2-dimethyltetrahydrothieno[3,4-d][1,3]dioxol-4-yl)-9H-purine (37 mg, 0.101 mmol) and 80% HCOOH (2 mL) were used to obtain a title compound as a white solid (25 mg, 76%).
¹H NMR (400 MHz, DMSO-d₆) *δ* 9.06 (s, 1H), 5.96 (d, *J* = 7.2 Hz, 1H), 4.62 (dd, *J* = 7.2, 3.1 Hz, 1H), 4.35 (d, *J* = 2.9 Hz, 1H), 3.45 (dd, *J* = 10.7, 4.0 Hz, 1H), 2.83 (dd, *J* = 10.8, 2.7 Hz, 1H), 2.55 - 2.51 (m, 2H), 1.21 (t, *J* = 7.5 Hz, 3H); LCMS m/z 325 [M+H]+

### <Preparation Example 5> Preparation of (2R,3R,4S)-2-(6-chloro-2-(pent-1-yn-1-yl)-9H-purin-9-yl)tetrahydrothiophene-3,4-diol

### Step 1: Preparation of 6-chloro-9-((3aR,4R,6aS)-2,2-dimethyltetrahydrothieno[3,4-d][1,3]dioxol-4-yl)-2-(pent-1-yn-1-yl)-9H-purine

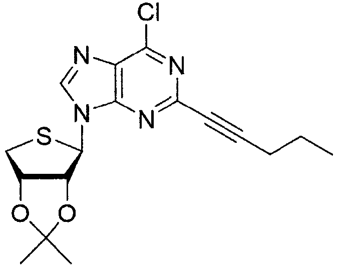

By using the same method as in Step 1 of Preparation Example 3, 1-pentyne (0.17 mL, 1.71 mmol), DMF (7.7 mL), Pd(PPh₃)₄ 328 mg (0.284 mmol), CuI (26 mg, 0.137 mmol) and CS₂CO₃ (372 mg, 1.14 mmol) were used to obtain a title compound as a yellow solid (265 mg, 61%).
¹H NMR (400 MHz, CDCl₃) *δ* 8.16 (s, 1H), 5.90 (s, 1H), 5.33 (t, *J* = 4.7 Hz, 1H), 5.23 (d, *J* = 5.4 Hz, 1H), 3.81 (dd, *J* = 12.9, 4.4 Hz, 1H), 3.25 (d, *J* = 12.8 Hz, 1H), 2.47 (t, *J* = 7.1 Hz, 2H), 1.79 - 1.64 (m, 2H), 1.59 (s, 3H), 1.37 (s, 3H), 1.08 (t, *J* = 7.4 Hz, 3H); LCMS m/z 379 [M+H]+

### Step 2: Preparation of (2R,3R,4S)-2-(6-chloro-2-(pent-1-yn-1-yl)-9H-purin-9-yl)tetrahydrothiophene-3,4-diol

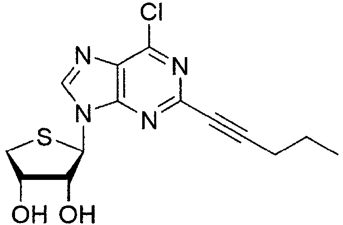

By using the same method as Step 2 of Preparation Example 3, 6-chloro-9-((3aR,4R,6aS)-2,2-dimethyltetrahydrothieno[3,4-d][1,3]dioxol-4-yl)-2-(pent-1-yn-1-yl)-9H-purine (265 mg, 0.7 mmol) and 80% HCOOH (14 mL) were used to obtain a title compound as a white solid.
¹H NMR (400 MHz, DMSO-d₆) *δ* 9.06 (s, 1H), 5.96 (d, *J* = 7.2 Hz, 1H), 5.63 (d, *J* = 5.9 Hz, 1H), 5.44 (d, *J* = 4.1 Hz, 1H), 4.62 (td, *J* = 7.2, 3.4 Hz, 1H), 4.39 - 4.32 (m, 1H), 3.45 (dd, *J =* 10.8, 4.0 Hz, 1H), 2.82 (dd, *J =* 10.7, 2.6 Hz, 1H), 2.50 - 2.47 (m, 2H), 1.68 - 1.54 (m, 2H), 1.02 (t, *J =* 7.4 Hz, 3H); LCMS m/z 339 [M+H]+

### <Preparation Example 6> Preparation of (2R,3R,4S)-2-(2,6-dichloro-9H-purin-9-yl)tetrahydrothiophene-3,4-diol

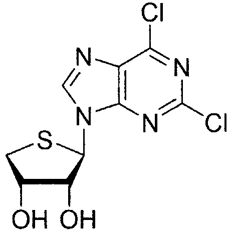

2,6-Dichloro-9-((3aR,4R,6aS)-2,2-dimethyltetrahydrothieno[3,4-d][1,3]dioxol-4-yl)-9H-purine (5.00 g, 14.4 mmol) was dissolved in 80% HCOOH (100 mL) and stirred at room temperature for 4 hours. The pH was adjusted to 8 with a saturated NaHCO₃ aqueous solution and extracted with EtOAc. The organic layer was dried over MgSO₄, filtered and concentrated. The residue obtained after concentration was filtered and dried to obtain a title compound as a white solid without further purification (3.23 g, 73%).
¹H NMR (400 MHz, CD₃OD) *δ* 8.88 (s, 1H), 6.08 (d, *J* = 6.6 Hz, 1H), 4.69 (dd, *J* = 6.4, 2.9 Hz, 1H), 4.48 (d, *J* = 3.3 Hz, 1H), 3.56 (dd, *J* = 10.9, 4.2 Hz, 1H), 2.97 (dd, *J* = 11.1, 2.8 Hz, 1H); LCMS m/z 307 [M+H]⁺

### [Example 1] (2R,3R,4S)-2-[6-[[(1R)-indan-1-yl]amino]-2-prop-1-ynyl-purin-9-yl]tetrahydrothiophene-3,4-diol

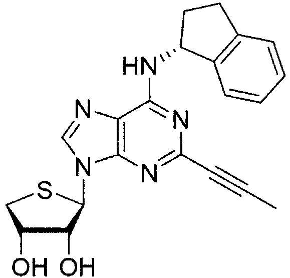

(2R,3R,4S)-2-(6-chloro-2-(prop-1-yn-1-yl)-9H-purin-9-yl)tetrahydrothiophene-3,4-diol (20 mg, 0.064 mmol) and (R)-(-)-1-Indanamine (9.4 mg, 0.071 mmol) were dissolved in DMF (0.6 mL), and DIPEA (13 µL, 0.071 mmol) was added. After stirring at room temperature for 4.5 days, DW was added to the reaction mixture, and the mixture was stirred for 30 minutes. The resulting solid was filtered and dried to obtain a title compound as a brown solid (12.3 mg, 47%).
¹H NMR (400 MHz, DMSO-d₆) *δ* 8.49 (s, 1H), 8.26 - 8.09 (m, 1H), 7.32 - 7.05 (m, 4H), 5.88 (d, *J =* 6.6 Hz, 2H), 5.56 (d, *J* = 6.3 Hz, 1H), 5.37 (d, *J* = 3.9 Hz, 1H), 4.63 (dd, *J* = 9.8, 6.8 Hz, 1H), 4.39 - 4.30 (m, 1H), 3.41 (dd, *J* = 10.8, 4.1 Hz, 1H), 3.05 - 2.95 (m, 1H), 2.90 - 2.77 (m, 2H), 2.45 - 2.38 (m, 1H), 2.18 - 2.06 (m, 1H), 2.03 (s, 3H); LCMS m/z 408 [M+H]⁺

### [Example 2] (2R,3R,4S)-2-[6-[[(1S)-indan-1-yl]amino]-2-prop-1-ynyl-purin-9-yl]tetrahydrothiophene-3,4-diol

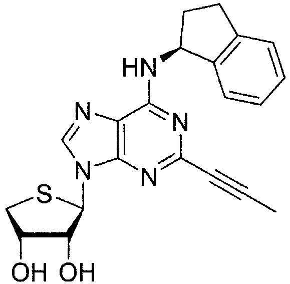

By using the same method as in Example 1, (2R,3R,4S)-2-(6-chloro-2-(prop-1-yn-1-yl)-9H-purin-9-yl)tetrahydrothiophene-3,4-diol (20 mg, 0.064 mmol), (S)-(+)-1-Indanamine (9.4 mg, 0.071 mmol), DMF (0.6 mL) and DIPEA (13 µL, 0.071 mmol) were used to obtain a title compound as an off-white solid (12.4 mg, 48%).
¹H NMR (400 MHz, DMSO-d₆) *δ* 8.49 (s, 1H), 8.27 - 8.08 (m, 1H), 7.31 - 7.06 (m, 4H), 5.88 (d, *J =* 6.7 Hz, 2H), 5.56 (d, *J =* 6.3 Hz, 1H), 5.37 (d, *J =* 4.2 Hz, 1H), 4.62 (br s, 1H), 4.35 (s, 1H), 3.41 (dd, *J* = 10.8, 4.0 Hz, 1H), 3.04 - 2.95 (m, 1H), 2.90 - 2.77 (m, 2H), 2.45 - 2.38 (m, 1H), 2.20 - 2.07 (m, 1H), 2.03 (s, 3H); LCMS m/z 408 [M+H]⁺

### [Example 3] (2R,3R,4S)-2-[2-but-1-ynyl-6-[[(1R)-indan-1-yl]amino]purin-9-yl]tetrahydrothiophene-3,4-diol

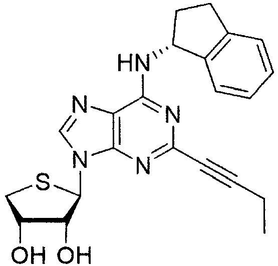

(2R,3R,4S)-2-(2-(but-1-yn-1-yl)-6-chloro-9H-purin-9-yl)tetrahydrothiophene-3,4-diol (30 mg, 0.092 mmol) and (R)-(-)-1-Indanamine (13.5 mg, 0.102 mmol) were dissolved in DMF (0.77 mL), and DIPEA (18.1 µL, 0.102 mmol) was added. The mixture was stirred at room temperature for 18 hours and at 50°C for 32 hours. DW was added to the reaction mixture and stirred for 30 minutes. The resulting solid was filtered and dried to obtain a title compound as an off-white solid (15 mg, 38%).
¹H NMR (400 MHz, DMSO-d₆) *δ* 8.51 (s, 1H), 8.25 - 8.10 (m, 1H), 7.30 - 7.07 (m, 4H), 5.89 (d, *J* = 7.3 Hz, 2H), 5.58 (br s, 1H), 5.40 (br s, 1H), 4.61 (br s, 1H), 4.35 (br s, 1H), 3.41 (dd, *J* = 10.9, 4.2 Hz, 1H), 3.05 - 2.94 (m, 1H), 2.91 - 2.77 (m, 2H), 2.46 - 2.38 (m, 3H), 2.22 - 1.93 (m, 1H), 1.17 (t, *J* = 7.5 Hz, 3H); LCMS m/z 422 [M+H]⁺

### [Example 4] (2R,3R,4S)-2-[2-but-1-ynyl-6-[[(1S)-indan-1-yl]amino]purin-9-yl]tetrahydrothiophene-3,4-diol

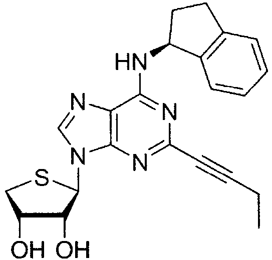

By using the same method as in Example 3, (2R,3R,4S)-2-(2-(but-1-yn-1-yl)-6-chloro-9H-purin-9-yl)tetrahydrothiophene-3,4-diol (30 mg, 0.092 mmol), (S)-(+)-1-Indanamine (13.5 mg, 0.102 mmol), DMF (0.77 mL) and DIPEA (18.1 µL, 0.102 mmol) were used to obtain a title compound as a white solid (15 mg, 38%).
¹H NMR (400 MHz, DMSO-d₆) *δ* 8.52 (s, 1H), 8.21 (br s, 1H), 7.35 - 7.03 (m, 4H), 5.98 - 5.83 (m, 2H), 5.65 - 5.51 (m, 1H), 5.47 - 5.31 (m, 1H), 4.61 (br s, 1H), 4.35 (s, 1H), 3.46 - 3.39 (m, 1H), 3.07 - 2.93 (m, 1H), 2.93 - 2.74 (m, 2H), 2.46 - 2.35 (m, 3H), 2.21 - 2.01 (m, 1H), 1.25 - 1.07 (m, 3H); LCMS m/z 422 [M+H]⁺

### [Example 5] (2R,3R,4S)-2-[6-[[(1R)-indan-1-yl]amino]-2-pent-1-ynyl-purin-9-yl]tetrahydrothiophene-3,4-diol

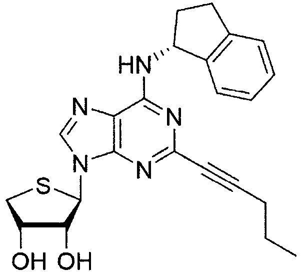

(2R,3R,4S)-2-(6-chloro-2-(pent-1-yn-1-yl)-9H-purin-9-yl)tetrahydrothiophene-3,4-diol (23 mg, 0.068 mmol) and (R)-(-)-1-indanamine (10 mg, 0.075 mmol) were dissolved in DMF (0.68 mL), DIPEA (14 µL, 0.075 mmol) was added, and the mixture was stirred at room temperature for 60 hours. DW was added to the reaction mixture, stirred for 30 minutes, and the resulting solid was filtered and dried. MeOH was added to the obtained solid, followed by filtering and drying to obtain a title compound as a white solid (1.4 mg, 4.7%).
¹H NMR (400 MHz, DMSO-d₆) *δ* 8.51 (s, 1H), 8.25 - 8.13 (m, 1H), 7.30 - 7.08 (m, 4H), 5.89 (d, *J =* 7.0 Hz, 2H), 5.57 (d, *J* = 5.8 Hz, 1H), 5.38 (d, *J* = 4.0 Hz, 1H), 4.65 - 4.57 (m, 1H), 4.38 - 4.32 (m, 1H), 3.41 (dd, *J* = 10.9, 4.2 Hz, 1H), 3.05 - 2.96 (m, 1H), 2.91 - 2.77 (m, 2H), 2.46 - 2.42 (m, 1H), 2.40 (t*, J* = 7.0 Hz, 2H), 2.18 - 2.06 (m, 1H), 1.57 (dd, *J* = 14.4, 7.4 Hz, 2H), 0.99 (t, *J* = 7.4 Hz, 3H); LCMS m/z 436 [M+H]⁺

### [Example 6] (2R,3R,4S)-2-[6-[[(1S)-indan-1-yl]amino]-2-pent-1-ynyl-purin-9-yl]tetrahydrothiophene-3,4-diol

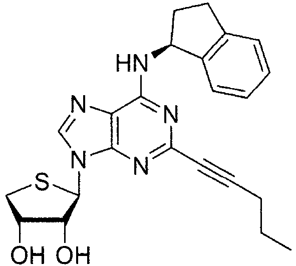

(2R,3R,4S)-2-(6-chloro-2-(pent-1-yn-1-yl)-9H-purin-9-yl)tetrahydrothiophene-3,4-diol (40 mg, 0.118 mmol) and (S)-(+)-1-indanamine (17.3 mg, 0.13 mmol) were dissolved in DMF (0.98 mL), and DIPEA (23.2 µL, 0.13 mmol) was added. After stirring at 50°C for 64 hours, DW was added to the reaction mixture and stirred for 30 minutes. The resulting solid was filtered and finally washed with DCM. The obtained solid was dried, and the filtrate was extracted with DCM, dried over MgSO₄, filtered and concentrated. The resulting residue was concentrated and purified by column chromatography (0-2% MeOH/DCM) and combined with the previously dried solid to obtain a title compound as an off-white solid (26 mg, 51%).
¹H NMR (400 MHz, DMSO-d₆) *δ* 8.51 (s, 1H), 8.25 - 8.09 (m, 1H), 7.33 - 7.00 (m, 4H), 5.90 (d, *J =* 7.1 Hz, 2H), 5.56 (d, *J =* 6.3 Hz, 1H), 5.37 (d, *J* = 4.1 Hz, 1H), 4.60 (dd, *J* = 9.6, 7.2 Hz, 1H), 4.39 - 4.29 (m, 1H), 3.42 (dd, *J* = 10.8, 4.0 Hz, 1H), 3.06 - 2.95 (m, 1H), 2.92 - 2.76 (m, 2H), 2.48 - 2.43 (m, 1H), 2.40 (t, *J =* 7.1 Hz, 2H), 2.21 - 2.06 (m, 1H), 1.57 (dd, *J* = 14.5, 7.2 Hz, 2H), 0.99 (t, *J* = 7.4 Hz, 3H); LCMS m/z 436 [M+H]⁺

### [Example 7] (2R,3R,4S)-2-[2-chloro-6-(indan-2-ylamino)purin-9-yl]tetrahydrothiophene-3,4-diol

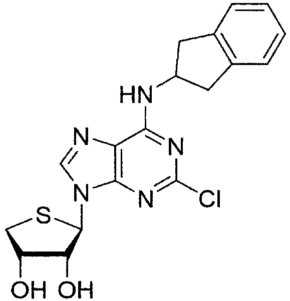

(2R,3R,4S)-2-(2,6-dichloro-9H-purin-9-yl)tetrahydrothiophene-3,4-diol (50 mg, 0.163 mmol) and 2-aminoindan (24 mg, 0.179 mmol) were dissolved in DMF (1.4 mL), and DIPEA (31 µL, 0.179 mmol) was added. After stirring at 50°C for 24 hours, DW was added to the reaction mixture, and stirred for 30 minutes. The resulting solid was filtered and dried to obtain a title compound as an off-white solid (17 mg, 26%).
¹H NMR (400 MHz, DMSO-d₆) *δ* 8.69 - 8.58 (m, 1H), 8.50 (s, 1H), 7.30 - 7.06 (m, 4H), 5.82 (d, *J* = 6.5 Hz, 1H), 5.58 (d, *J* = 6.1 Hz, 1H), 5.39 (d, *J* = 3.8 Hz, 1H), 4.88 (dd, *J* = 14.2, 6.9 Hz, 1H), 4.60 (td, *J* = 7.3, 3.3 Hz, 1H), 4.37 - 4.29 (m, 1H), 3.41 (dd, *J* = 10.8, 4.0 Hz, 1H), 3.26 (dd, *J* = 16.3, 8.1 Hz, 2H), 3.08 - 2.92 (m, 2H), 2.80 (dd, *J* = 10.8, 2.5 Hz, 1H); LCMS m/z 404 [M+H]⁺

### [Example 8] (2R,3R,4S)-2-[2-chloro-6-(indan-1-ylamino)purin-9-yl]tetrahydrothiophene-3,4-diol

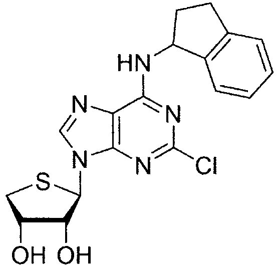

(2R,3R,4S)-2-(2,6-dichloro-9H-purin-9-yl)tetrahydrothiophene-3,4-diol (307 mg, 1.00 mmol) and 1-aminoindan (147 mg, 1.10 mmol) were dissolved in DMF (10 mL), and DIPEA (197 µL, 1.10 mmol) was added. After stirring at room temperature for 22 hours, DW was added to the reaction mixture, and stirred for 30 minutes. The resulting solid was filtered and dried to obtain a title compound as a gray solid (250 mg, 62%).
¹H NMR (400 MHz, DMSO-d₆) *δ* 8.71 (d, *J* = 8.0 Hz, 1H), 8.50 (s, 1H), 7.34 - 7.01 (m, 4H), 5.90 - 5.77 (m, 2H), 5.59 (d, *J* = 6.1 Hz, 1H), 5.40 (d, *J* = 3.4 Hz, 1H), 4.67 - 4.52 (m, 1H), 4.40 - 4.28 (m, 1H), 3.42 (dd, *J =* 11.0, 3.8 Hz, 1H), 3.07 - 2.95 (m, 1H), 2.91 - 2.76 (m, 2H), 2.47 - 2.38 (m, 1H), 2.22 - 2.07 (m, 1H); LCMS m/z 404 [M+H]⁺

### [Example 9] (2R,3R,4S)-2-[2-chloro-6-[[(1R)-indan-1-yl]amino]purin-9-yl]tetrahydrothiophene-3,4-diol

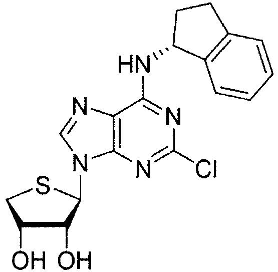

By using the same method as in Example 8, (2R,3R,4S)-2-(2,6-dichloro-9H-purin-9-yl)tetrahydrothiophene-3,4-diol (307 mg, 1.00 mmol), (R)-(-)-1-indanamine (147 mg, 1.10 mmol), DMF (10 mL) and DIPEA (197 µL, 1.10 mmol) were used to obtain a title compound as an off-white solid (300 mg, 74%).
¹H NMR (400 MHz, DMSO-d₆) *δ* 8.71 (d, *J =* 8.4 Hz, 1H), 8.50 (d, *J =* 5.9 Hz, 1H), 7.34 - 7.03 (m, 4H), 5.91 - 5.72 (m, 2H), 5.59 (d, *J =* 6.2 Hz, 1H), 5.39 (d, *J* = 4.0 Hz, 1H), 4.62 (dd, *J* = 9.6, 7.1 Hz, 1H), 4.35 (br s, 1H), 3.42 (dd, *J =* 10.8, 3.6 Hz, 1H), 3.07 - 2.96 (m, 1H), 2.91 - 2.76 (m, 2H), 2.48 - 2.39 (m, 1H), 2.21 - 2.06 (m, 1H); LCMS m/z 404 [M+H]⁺

### [Example 10] (2R,3R,4S)-2-[2-chloro-6-[[(1S)-indan-1-yl]amino]purin-9-yl]tetrahydrothiophene-3,4-diol

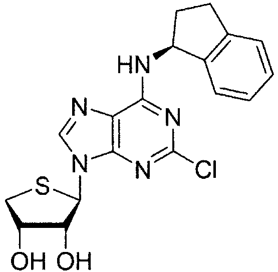

By using the same method as in Example 7, (2R,3R,4S)-2-(2,6-dichloro-9H-purin-9-yl)tetrahydrothiophene-3,4-diol (50 mg, 0.163 mmol), (S)-(+)-1-indanamine (24 mg, 0.179 mmol), DMF (1.4 mL) and DIPEA (31 µL, 0.179 mmol) were used to obtain a title compound as a white solid (41 mg, 62%).
¹H NMR (400 MHz, DMSO-d₆) *δ* 8.71 (d, *J* = 8.2 Hz, 1H), 8.50 (s, 1H), 7.31 - 7.08 (m, 4H), 5.89 - 5.75 (m, 2H), 5.59 (d, *J =* 6.0 Hz, 1H), 5.40 (d, *J =* 4.0 Hz, 1H), 4.61 (br s, 1H), 4.34 (s, 1H), 3.42 (dd, *J =* 10.8, 3.9 Hz, 1H), 3.07 - 2.96 (m, 1H), 2.90 - 2.77 (m, 2H), 2.47 - 2.39 (m, 1H), 2.21 - 2.06 (m, 1H); LCMS m/z 404 [M+H]⁺

### [Example 11] (2R,3R,4S)-2-[2-chloro-6-[[(1R)-4-methoxyindan-1-yl]amino]purin-9-yl]tetrahydrothiophene-3,4-diol

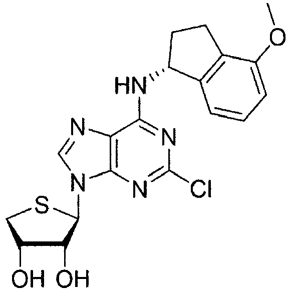

(2R,3R,4S)-2-(2,6-dichloro-9H-purin-9-yl)tetrahydrothiophene-3,4-diol (30 mg, 0.098 mmol) and (1R)-4-methoxy-2,3-dihydro-1H-inden-1-amine hydrochloride (21.5 mg, 0.107 mmol) were dissolved in DMF (0.98 mL), and DIPEA (37 µL, 0.215 mmol) was added. After stirring at 50°C for 24 hours, DW was added to the reaction mixture, and stirred for 30 minutes. The resulting solid was filtered and dried. The obtained solid was dissolved in 0.5 mL of DCM, 3 mL of hexane was added, and the mixture was stirred at room temperature for 30 minutes. The resulting solid was filtered and dried to obtain a title compound as a brown solid (18 mg, 42%).
¹H NMR (400 MHz, DMSO-d₆) *δ* 8.69 (d, *J* = 8.5 Hz, 1H), 8.50 (d, *J =* 9.0 Hz, 1H), 7.13 (t, *J* = 7.7 Hz, 1H), 6.81 (dd, *J* = 14.8, 7.9 Hz, 2H), 5.89 - 5.74 (m, 2H), 5.59 (d, *J* = 6.5 Hz, 1H), 5.40 (d, *J =* 3.7 Hz, 1H), 4.67 - 4.54 (m, 1H), 4.34 (s, 1H), 3.79 (s, 3H), 3.41 (dd, *J =* 10.7, 3.3 Hz, 1H), 3.02 - 2.88 (m, 1H), 2.80 (dd, *J =* 10.9, 2.5 Hz, 1H), 2.76 - 2.62 (m, 1H), 2.47 - 2.37 (m, 1H), 2.18 - 2.02 (m, 1H); LCMS m/z 434 [M+H]⁺

### [Example 12] (2R,3R,4S)-2-[2-chloro-6-[[(1R)-5-methoxyindan-1-yl]amino]purin-9-yl]tetrahydrothiophene-3,4-diol

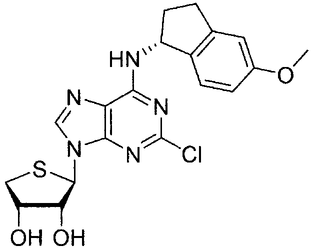

By using the same method as in Example 11, (2R,3R,4S)-2-(2,6-dichloro-9H-purin-9-yl)tetrahydrothiophene-3,4-diol (30 mg, 0.098 mmol), (1R)-5-methoxy-2,3-dihydro-1H-inden-1-amine hydrochloride (21.5 mg, 0.107 mmol), DMF (0.98 mL) and DIPEA (37 µL, 0.215 mmol) were used to obtain a title compound as an off-white solid (19 mg, 45%).
¹H NMR (400 MHz, DMSO-d₆) *δ* 8.63 (d, *J* = 7.7 Hz, 1H), 8.50 (d, *J =* 11.0 Hz, 1H), 7.07 (dd, *J =* 32.9, 8.0 Hz, 1H), 6.83 (s, 1H), 6.70 (d, *J* = 7.8 Hz, 1H), 5.83 (d, *J =* 7.5 Hz, 1H), 5.72 (dd, *J =* 15.3, 7.1 Hz, 1H), 5.59 (d, *J* = 6.0 Hz, 1H), 5.40 (d, *J* = 3.9 Hz, 1H), 4.65 - 4.55 (m, 1H), 4.34 (s, 1H), 3.72 (s, 3H), 3.41 (dd, *J =* 11.0, 3.6 Hz, 1H), 3.06 - 2.92 (m, 1H), 2.87 - 2.71 (m, 2H), 2.44 - 2.36 (m, 1H), 2.19 - 2.06 (m, 1H); LCMS m/z 434 [M+H]⁺

### [Example 13] (2R,3R,4S)-2-[2-chloro-6-[[(1R)-6-methoxyindan-1-yl]amino]purin-9-yl]tetrahydrothiophene-3,4-diol

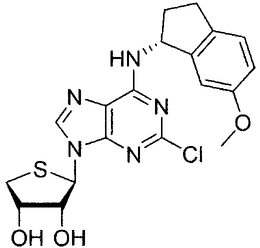

By using the same method as in Example 11, (2R,3R,4S)-2-(2,6-dichloro-9H-purin-9-yl)tetrahydrothiophene-3,4-diol (60 mg, 0.195 mmol), (1R)-6-methoxy-2,3-dihydro-1H-inden-1-amine hydrochloride (43 mg, 0.215 mmol), DMF (1.95 mL) and DIPEA (74 µL, 0.43 mmol) were used to obtain a title compound as an off-white solid (45 mg, 53%).
¹H NMR (400 MHz, DMSO-d₆) *δ* 8.70 (d, *J =* 8.3 Hz, 1H), 8.51 (d, *J =* 8.1 Hz, 1H), 7.16 (d, *J =* 9.0 Hz, 1H), 6.84 - 6.67 (m, 2H), 5.83 (d, *J =* 7.0 Hz, 1H), 5.74 (dd, *J =* 15.2, 8.3 Hz, 1H), 5.59 (d, *J* = 6.2 Hz, 1H), 5.41 (s, 1H), 4.61 (dd, *J* = 9.4, 7.3 Hz, 1H), 4.34 (s, 1H), 3.66 (s, 3H), 3.42 (dd, *J =* 10.9, 4.4 Hz, 1H), 3.00 - 2.88 (m, 1H), 2.85 - 2.70 (m, 2H), 2.47 - 2.38 (m, 1H), 2.20 - 2.05 (m, 1H); LCMS m/z 434 [M+H]⁺

### [Example 14] (2R,3R,4S)-2-[2-chloro-6-[[(1R)-7-methoxyindan-1-yl]amino]purin-9-yl]tetrahydrothiophene-3,4-diol

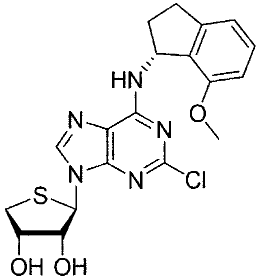

By using the same method as in Example 11, (2R,3R,4S)-2-(2,6-dichloro-9H-purin-9-yl)tetrahydrothiophene-3,4-diol (60 mg, 0.195 mmol), (1R)-7-methoxy-2,3-dihydro-1H-inden-1-amine hydrochloride (43 mg, 0.215 mmol), DMF (1.95 mL) and DIPEA (74 µL, 0.43 mmol) were used to obtain a title compound as an off-white solid (80 mg, 94%).
¹H NMR (400 MHz, DMSO-d₆) *δ* 8.55 - 8.29 (m, 2H), 7.23 (t, *J* = 7.8 Hz, 1H), 6.86 (d, *J =* 7.5 Hz, 1H), 6.77 (d, *J =* 8.1 Hz, 1H), 5.82 (d, *J =* 7.1 Hz, 2H), 5.58 (d, *J* = 5.5 Hz, 1H), 5.40 (s, 1H), 4.67 - 4.53 (m, 1H), 4.34 (s, 1H), 3.59 (d, *J =* 23.9 Hz, 3H), 3.41 (dd, *J* = 10.6, 3.3 Hz, 1H), 3.19 - 3.01 (m, 1H), 2.87 - 2.71 (m, 2H), 2.48 - 2.36 (m, 1H), 2.05 - 1.91 (m, 1H); LCMS m/z 434 [M+H]⁺

### [Example 15] (2R,3R,4S)-2-[2-chloro-6-[[(1R)-4-fluoroindan-1-yl]amino]purin-9-yl]tetrahydrothiophene-3,4-diol

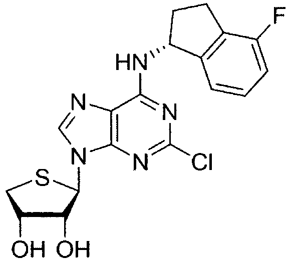

By using the same method as in Example 11, (2R,3R,4S)-2-(2,6-dichloro-9H-purin-9-yl)tetrahydrothiophene-3,4-diol (60 mg, 0.195 mmol), (R)-4-Fluoroindan-1-amine hydrochloride (40.3 mg, 0.215 mmol), DMF (1.95 mL) and DIPEA (74 µL, 0.43 mmol) were used to obtain a title compound as an off-white solid (44 mg, 54%).
¹H NMR (400 MHz, DMSO-d₆) *δ* 8.78 (d, *J* = 7.8 Hz, 1H), 8.52 (d, *J* = 8.4 Hz, 1H), 7.20 (dd, *J =* 13.2, 7.6 Hz, 1H), 7.11 - 6.95 (m, 2H), 5.83 (d, *J =* 6.4 Hz, 2H), 5.59 (d, *J =* 6.2 Hz, 1H), 5.40 (d, *J =* 4.0 Hz, 1H), 4.61 (dd, *J =* 9.9, 7.4 Hz, 1H), 4.39 - 4.30 (m, 1H), 3.42 (dd, *J =* 11.1, 4.0 Hz, 1H), 3.13 - 3.01 (m, 1H), 2.92 - 2.76 (m, 2H), 2.47 - 2.41 (m, 1H), 2.25 - 2.10 (m, 1H); LCMS m/z 422 [M+H]⁺

### [Example 16] (2R,3R,4S)-2-[2-chloro-6-[[(1R)-5-fluoroindan-1-yl]amino]purin-9-yl]tetrahydrothiophene-3,4-diol

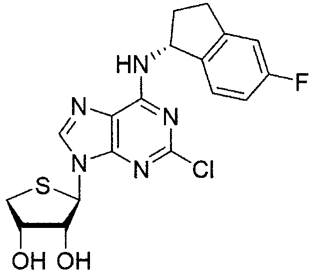

By using the same method as in Example 11, (2R,3R,4S)-2-(2,6-dichloro-9H-purin-9-yl)tetrahydrothiophene-3,4-diol (60 mg, 0.195 mmol), (R)-5-fluoroindan-1-amine hydrochloride (40.3 mg, 0.215 mmol), DMF (1.95 mL) and DIPEA (74 µL, 0.43 mmol) were used to obtain a title compound as an off-white solid (33 mg, 40%).
¹H NMR (400 MHz, DMSO-d₆) *δ* 8.73 (d, *J* = 8.4 Hz, 1H), 8.51 (d, *J* = 8.8 Hz, 1H), 7.21 (dd, *J* = 7.2, 5.7 Hz, 1H), 7.10 (d, *J* = 8.7 Hz, 1H), 6.95 (t, *J* = 8.8 Hz, 1H), 5.83 (d, *J* = 7.3 Hz, 1H), 5.75 (dd, *J* = 16.5, 8.0 Hz, 1H), 5.59 (d, *J* = 5.8 Hz, 1H), 5.40 (d, *J =* 3.0 Hz, 1H), 4.61 (td, *J =* 6.9, 3.2 Hz, 1H), 4.34 (s, 1H), 3.41 (dd, *J* = 10.8, 3.9 Hz, 1H), 3.09 - 2.97 (m, 1H), 2.91 - 2.76 (m, 2H), 2.48 - 2.40 (m, 1H), 2.23 - 2.10 (m, 1H); LCMS m/z 422 [M+H]⁺

### [Example 17] (2R,3R,4S)-2-[2-chloro-6-[[(1R)-4-chloroindan-1-yl]amino]purin-9-yl]tetrahydrothiophene-3,4-diol

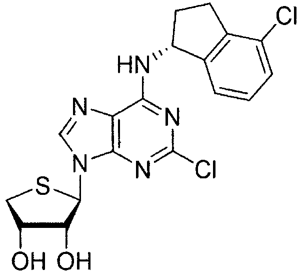

By using the same method as in Example 11, (2R,3R,4S)-2-(2,6-dichloro-9H-purin-9-yl)tetrahydrothiophene-3,4-diol (30 mg, 0.098 mmol), (1R)-4-chloro-2,3-dihydro-1H-inden-1-amine hydrochloride (21.5 mg, 0.107 mmol), DMF (0.98 mL) and DIPEA (37 µL, 0.215 mmol) were used to obtain a title compound as a gray solid (18.5 mg, 43%).
¹H NMR (400 MHz, DMSO-d₆) *δ* 8.82 (d, *J =* 8.9 Hz, 1H), 8.52 (d, *J =* 8.6 Hz, 1H), 7.30 (d, *J =* 6.1 Hz, 1H), 7.25 - 7.06 (m, 2H), 5.94 - 5.79 (m, 2H), 5.60 (d, *J* = 6.4 Hz, 1H), 5.41 (d, *J =* 3.7 Hz, 1H), 4.61 (dd, *J =* 9.3, 7.4 Hz, 1H), 4.34 (s, 1H), 3.42 (dd, *J =* 11.0, 3.7 Hz, 1H), 3.12 - 2.99 (m, 1H), 2.94 - 2.76 (m, 2H), 2.49 - 2.44 (m, 1H), 2.23 - 2.09 (m, 1H); LCMS m/z 438 [M+H]⁺

### [Example 18] (2R,3R,4S)-2-[2-chloro-6-[[(1R)-5-chloroindan-1-yl]amino]purin-9-yl]tetrahydrothiophene-3,4-diol

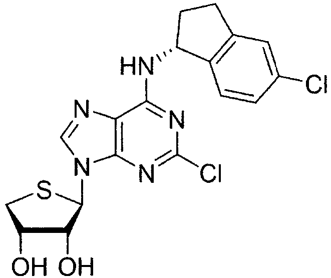

By using the same method as in Example 11, (2R,3R,4S)-2-(2,6-dichloro-9H-purin-9-yl)tetrahydrothiophene-3,4-diol (60 mg, 0.195 mmol), (R)-5-chloro-2,3-dihydro-1H-inden-1-amine (36 mg, 0.215 mmol), DMF (1.95 mL) and DIPEA (37 µL, 0.215 mmol) were used to obtain a title compound as an off-white solid (19.8 mg, 21%).
¹H NMR (400 MHz, DMSO-d₆) *δ* 8.75 (d, *J =* 7.9 Hz, 1H), 8.51 (s, 1H), 7.34 (s, 1H), 7.26 - 7.09 (m, 2H), 5.83 (d, *J =* 7.0 Hz, 1H), 5.76 (dd, *J =* 16.4, 7.9 Hz, 1H), 5.58 (d, *J* = 6.2 Hz, 1H), 5.39 (d, *J =* 3.6 Hz, 1H), 4.61 (td, *J =* 7.0, 3.3 Hz, 1H), 4.39 - 4.30 (m, 1H), 3.42 (dd, *J* = 11.0, 4.0 Hz, 1H), 3.07 - 2.96 (m, 1H), 2.92 - 2.76 (m, 2H), 2.47 - 2.40 (m, 1H), 2.23 - 2.08 (m, 1H); LCMS m/z 438 [M+H]⁺

### [Example 19] (2R,3R,4S)-2-[2-chloro-6-[[(1R)-4-bromoindan-1-yl]amino]purin-9-yl]tetrahydrothiophene-3,4-diol

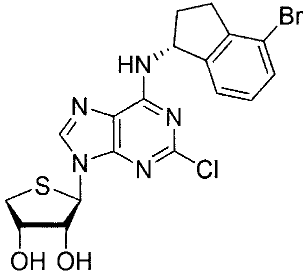

By using the same method as in Example 11, (2R,3R,4S)-2-(2,6-dichloro-9H-purin-9-yl)tetrahydrothiophene-3,4-diol (76.2 mg, 0.248 mmol), (R)-4-bromo-2,3-dihydro-1H-inden-1-amine (58 mg, 0.271 mmol), DMF (2.5 mL) and DIPEA (47 µL, 0.271 mmol) were used to obtain a title compound as a brown solid (54 mg, 45%).
¹H NMR (400 MHz, DMSO-d₆) δ 8.82 (d, *J =* 8.2 Hz, 1H), 8.52 (d, *J =* 7.8 Hz, 1H), 7.45 (d, *J =* 7.6 Hz, 1H), 7.21 (d, *J =* 7.4 Hz, 1H), 7.11 (t, *J =* 7.7 Hz, 1H), 5.95 - 5.80 (m, 2H), 5.59 (d, *J =* 6.3 Hz, 1H), 5.40 (d, *J =* 3.8 Hz, 1H), 4.61 (td, *J =* 7.2, 3.3 Hz, 1H), 4.40 - 4.29 (m, 1H), 3.42 (dd, *J =* 10.9, 4.0 Hz, 1H), 3.09 - 2.95 (m, 1H), 2.92 - 2.75 (m, 2H), 2.49 - 2.43 (m, 1H), 2.22 - 2.09 (m, 1H); LCMS m/z 482 [M+H]⁺

### [Example 20] (2R,3R,4S)-2-[2-chloro-6-[[(1R)-5-bromoindan-1-yl]amino]purin-9-yl]tetrahydrothiophene-3,4-diol

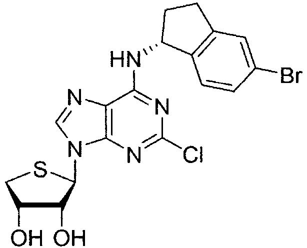

By using the same method as in Example 11, (2R,3R,4S)-2-(2,6-dichloro-9H-purin-9-yl)tetrahydrothiophene-3,4-diol (30 mg, 0.098 mmol), (R)-5-bromo-2,3-dihydro-1H-inden-1-amine (22.7 mg, 0.107 mmol), DMF (0.98 mL) and DIPEA (18.3 µL, 0.107 mmol) were used to obtain a title compound as a white solid (28 mg, 59%).
¹H NMR (400 MHz, DMSO-d₆) δ 8.76 (d, *J* = 7.7 Hz, 1H), 8.51 (br s, 1H), 7.48 (s, 1H), 7.32 (d, *J =* 8.3 Hz, 1H), 7.11 (dd*, J =* 23.4, 8.5 Hz, 1H), 5.83 (d, *J =* 7.0 Hz, 1H), 5.75 (dd, *J =* 16.2, 7.8 Hz, 1H), 5.59 (d, *J =* 6.3 Hz, 1H), 5.40 (d, *J* = 3.6 Hz, 1H), 4.67 - 4.55 (m, 1H), 4.34 (s, 1H), 3.42 (dd, *J =* 10.7, 3.7 Hz, 1H), 3.08 - 2.96 (m, 1H), 2.92 - 2.75 (m, 2H), 2.47 - 2.38 (m, 1H), 2.21 - 2.09 (m, 1H); LCMS m/z 482 [M+H]⁺

### [Example 21] (2R,3R,4S)-2-[2-chloro-6-[[(1R)-6-bromoindan-1-yl]amino]purin-9-yl]tetrahydrothiophene-3,4-diol

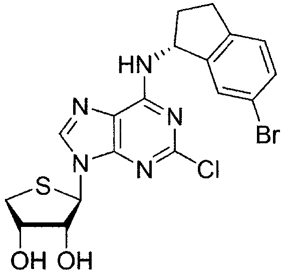

By using the same method as in Example 11, (2R,3R,4S)-2-(2,6-dichloro-9H-purin-9-yl)tetrahydrothiophene-3,4-diol (30 mg, 0.098 mmol), (R)-6-bromo-2,3-dihydro-1H-inden-1-amine (22.7 mg, 0.107 mmol), DMF (0.98 mL) and DIPEA (18.3 µL, 0.107 mmol) were used to obtain a title compound as a white solid (33 mg, 70%).
¹H NMR (400 MHz, DMSO-d₆) *δ* 8.80 (d, *J =* 8.8 Hz, 1H), 8.52 (d, *J =* 7.6 Hz, 1H), 7.40 (d, *J =* 7.3 Hz, 1H), 7.34 (s, 1H), 7.24 (d*, J =* 7.7 Hz, 1H), 5.81 (dd, *J =* 20.2, 7.9 Hz, 2H), 5.59 (d, *J =* 6.5 Hz, 1H), 5.40 (d, *J =* 3.7 Hz, 1H), 4.62 (td, *J =* 7.0, 3.3 Hz, 1H), 4.39 - 4.30 (m, 1H), 3.42 (dd, *J =* 11.0, 4.0 Hz, 1H), 3.04 - 2.93 (m, 1H), 2.87 - 2.74 (m, 2H), 2.47 - 2.39 (m, 1H), 2.21 - 2.07 (m, 1H); LCMS m/z 482 [M+H]⁺

### [Example 22] (2R,3R,4S)-2-[2-chloro-6-[[(1R)-4-methylindan-1-yl]amino]purin-9-yl]tetrahydrothiophene-3,4-diol

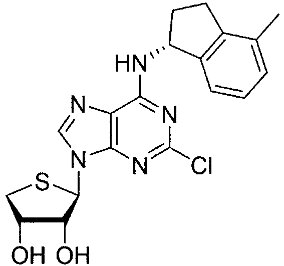

By using the same method as in Example 11, (2R,3R,4S)-2-(2,6-dichloro-9H-purin-9-yl)tetrahydrothiophene-3,4-diol (30 mg, 0.098 mmol), (R)-4-methyl-indan-1-ylamine hydrochloride (21.5 mg, 0.107 mmol), DMF (0.98 mL) and DIPEA (37 µL, 0.215 mmol) were used to obtain a title compound as a brown solid (22 mg, 54%).
¹H NMR (400 MHz, DMSO-d₆) δ 8.68 (d, *J* = 8.1 Hz, 1H), 8.50 (d, *J* = 6.8 Hz, 1H), 7.13 - 6.89 (m, 3H), 5.89 - 5.75 (m, 2H), 5.65 - 5.55 (m, 1H), 5.40 (d, *J =* 4.0 Hz, 1H), 4.61 (t, *J* = 8.4 Hz, 1H), 4.34 (s, 1H), 3.42 (dd, *J =* 10.8, 3.8 Hz, 1H), 3.03 - 2.91 (m, 1H), 2.85 - 2.69 (m, 2H), 2.47 - 2.39 (m, 1H), 2.24 (s, 3H), 2.17 - 2.03 (m, 1H); LCMS m/z 418 [M+H]⁺

### [Example 23] (2R,3R,4S)-2-[2-chloro-6-[[(1R)-4-(trifluoromethyl)indan-1-yl]amino]purin-9-yl]tetrahydrothiophene-3,4-diol

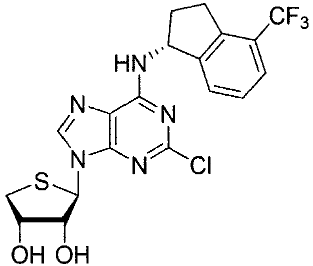

By using the same method as in Example 11, (2R,3R,4S)-2-(2,6-dichloro-9H-purin-9-yl)tetrahydrothiophene-3,4-diol (30 mg, 0.098 mmol), (1R)-4-(trifluoromethyl)-2,3-dihydro-1H-inden-1-amine (22 mg, 0.107 mmol), DMF (0.98 mL) and DIPEA (18.3 µL, 0.107 mmol) were used to obtain a title compound as an off-white solid (18 mg, 39%).
¹H NMR (400 MHz, DMSO-d₆) *δ* 8.82 (d, *J* = 8.1 Hz, 1H), 8.52 (br s, 1H), 7.58 (d, *J* = 7.8 Hz, 1H), 7.50 (d, *J =* 7.3 Hz, 1H), 7.38 (t, *J =* 7.4 Hz, 1H), 5.85 (t, *J* = 7.7 Hz, 2H), 5.60 (d, *J =* 5.0 Hz, 1H), 5.42 (d, *J =* 3.5 Hz, 1H), 4.61 (br s, 1H), 4.34 (s, 1H), 3.42 (dd, *J =* 10.5, 3.8 Hz, 1H), 3.25 - 3.13 (m, 1H), 3.06 - 2.94 (m, 1H), 2.86 - 2.75 (m, *J =* 10.5 Hz, 1H), 2.58 - 2.52 (m, 1H), 2.27 - 2.13 (m, 1H); LCMS m/z 472 [M+H]⁺

### [Example 24] (1R)-1-[[2-chloro-9-[(2R,3R,4S)-3,4-dihydroxytetrahydrothiophen-2-yl]purin-6-yl]amino]indane-4-carbonitrile

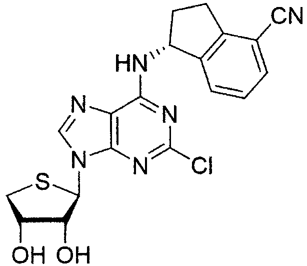

By using the same method as in Example 11, (2R,3R,4S)-2-(2,6-dichloro-9H-purin-9-yl)tetrahydrothiophene-3,4-diol (30 mg, 0.098 mmol), (R)-1-amino-2,3-dihydro-1H-indene-4-carbonitrile hydrochloride (21 mg, 0.107 mmol), DMF (0.98 mL) and DIPEA (37 µL, 0.215 mmol) were used to obtain a title compound as a white solid (22 mg, 52%).
¹H NMR (400 MHz, DMSO-d₆) *δ* 8.85 (d, *J* = 8.1 Hz, 1H), 8.52 (br s, 1H), 7.70 (d, *J =* 7.7 Hz, 1H), 7.51 (dd, J = 23.4, 8.0 Hz, 1H), 7.36 (t, J = 7.6 Hz, 1H), 5.93 - 5.79 (m, 2H), 5.60 (d, *J* = 5.6 Hz, 1H), 5.42 (s, 1H), 4.61 (br s, 1H), 4.34 (s, 1H), 3.42 (dd, *J* = 10.5, 3.9 Hz, 1H), 3.22 - 3.11 (m, 1H), 3.10 - 2.97 (m, 1H), 2.80 (d, *J* = 10.3 Hz, 1H), 2.59 - 2.52 (m, 1H), 2.27 - 2.13 (m, 1H); LCMS m/z 429 [M+H]⁺

### [Example 25] (R)-1-((2-chloro-9-((2R,3R,4S)-3,4-dihydroxytetrahydrothiophen-2-yl)-9H-purin-6-yl)amino)-2,3-dihydro-1H-indene-4-carboxamide

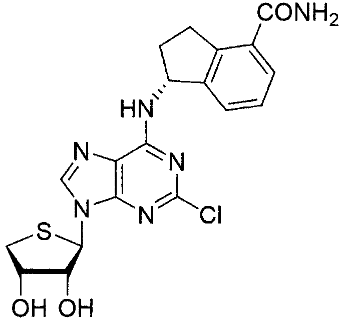

After dissolving (1R)-1-[[2-chloro-9-[(2R,3R,4S)-3,4-dihydroxytetrahydrothiophen-2-yl]purin-6-yl]amino]indane-4-carbonitrile (10 mg, 0.023 mmol) in DMSO (0.1 mL), K₂CO₃ (5.7 mg, 0.042 mmol) and H₂O₂ (35% in H₂O, 33 µL) were added. After stirring at room temperature for 2 hours, the mixture was diluted with EtOAc and washed with DW. The organic layer was dried over MgSO₄, filtered and concentrated, and the resulting residue was purified by prep TLC (5% MeOH/DCM) to obtain a title compound as a white solid (3.2 mg, 32%).
¹H NMR (400 MHz, DMSO-d₆) *δ* 8.75 (d, *J =* 8.5 Hz, 1H), 8.50 (s, 1H), 7.72 (s, 1H), 7.52 (d, *J =* 7.4 Hz, 1H), 7.30 (d, *J =* 8.2 Hz, 2H), 7.24 - 7.17 (m, 1H), 5.90 - 5.74 (m, 2H), 5.59 (d, *J =* 5.7 Hz, 1H), 5.40 (d, *J =* 3.6 Hz, 1H), 4.67 - 4.56 (m, 1H), 4.34 (s, 1H), 3.42 (dd, *J =* 10.6, 4.0 Hz, 1H), 3.29 - 3.22 (m, 1H), 3.09 -2.95 (m, 1H), 2.80 (dd, *J =* 11.1, 2.2 Hz, 1H), 2.47 -2.37 (m, 1H), 2.18 -2.02 (m, 1H); LCMS m/z 447 [M+H]⁺

### [Example 26] (2R,3R,4S)-2-[2-chloro-6-[[(1R)-7-bromoindan-1-yl]amino]purin-9-yl]tetrahydrothiophene-3,4-diol

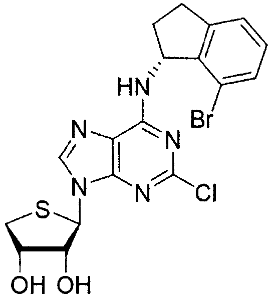

After dissolving (2R,3R,4S)-2-(2,6-dichloro-9H-purin-9-yl)tetrahydrothiophene-3,4-diol (60 mg, 0.195 mmol) and (1R)-7-bromoindanylamine (45.6 mg, 0.215 mmol) in 1,4-dioxane (2 mL), DIPEA (37 µL, 0.215 mmol) was added. After stirring at 50°C for 42.5 hours, the reaction mixture was concentrated under reduced pressure. DCM was added to the obtained solid, followed by filtration and drying to obtain a title compound as a white solid (54 mg, 57%).
¹H NMR (400 MHz, DMSO-d6) δ 8.75 (d, J *=* 8.9 Hz, 1H), 8.45 (s, 1H), 7.39 (d, J = 7.3 Hz, 1H), 7.30 (d, J = 7.1 Hz, 1H), 7.21 (t, J = 7.6 Hz, 1H), 5.82 (d, J = 7.0 Hz, 2H), 5.64 - 5.53 (m, 1H), 5.45 - 5.34 (m, 1H), 4.61 (s, 1H), 4.34 (s, 1H), 3.41 (dd, J = 10.4, 3.8 Hz, 1H), 3.29 - 3.14 (m, 1H), 2.95 -2.84 (m, 1H), 2.80 (d, J = 10.8 Hz, 1H), 2.48 -2.41 (m, 1H), 2.10 - 1.93 (m, 1H); LCMS m/z 482 [M+H]+

### [Example 27] (2R,3R,4S)-2-[2-chloro-6-[[(1R)-5-methylindan-1-yl]amino]purin-9-yl]tetrahydrothiophene-3,4-diol

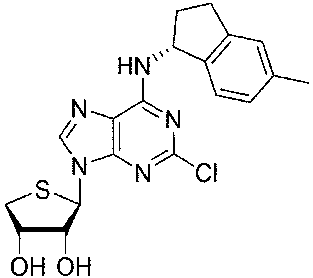

By using the same method as in Example 26, (2R,3R,4S)-2-(2,6-dichloro-9H-purin-9-yl)tetrahydrothiophene-3,4-diol (60 mg, 0.195 mmol), (1R)-5-methyl-2,3-dihydro-1H-indan-1-amine (32 mg, 0.215 mmol), DMF (1.95 mL) and DIPEA (37 µL, 0.215 mmol) were used to obtain a title compound as a white solid (63 mg, 77%).
¹H NMR (400 MHz, DMSO-d6) *δ* 8.66 (d, *J =* 8.4 Hz, 1H), 8.49 (s, 1H), 7.07 (s, 2H), 6.94 (d, *J* = 7.5 Hz, 1H), 5.89 - 5.80 (m, 1H), 5.75 (dd, *J* = 16.0, 7.6 Hz, 1H), 5.66 - 5.54 (m, 1H), 5.41 (s, 1H), 4.62 (s, 1H), 4.34 (s, 1H), 3.41 (dd, *J* = 10.9, 4.0 Hz, 1H), 3.03 -2.91 (m, 1H), 2.88 -2.73 (m, 2H), 2.47 -2.36 (m, 1H), 2.28 (s, 3H), 2.17 - 1.91 (m, 1H); LCMS m/z 418 [M+H]+

### [Example 28] (2R,3R,4S)-2-[2-chloro-6-[[(1R)-5-(trifluoromethyl)indan-1-yl]amino]purin-9-yl]tetrahydrothiophene-3,4-diol

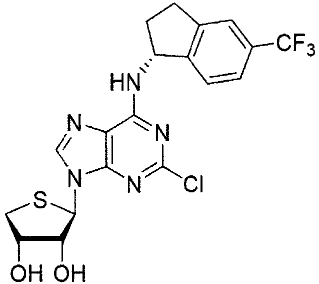

After dissolving (2R,3R,4S)-2-(2,6-dichloro-9H-purin-9-yl)tetrahydrothiophene-3,4-diol (60 mg, 0.195 mmol) and (R)-5-(Ttifluoromethyl)-2,3-dihydro-1H-inden-1-amine hydrochloride (43.3 mg, 0.215 mmol) in 1,4-dioxane (2 mL), DIPEA (74 µL, 0.43 mmol) was added, and the mixture was stirred at 50°C for 42.5 hours. The reaction mixture was concentrated under reduced pressure, and the resulting residue was purified by column chromatography (0-1% MeOH in DCM) and recrystallized from DCM to obtain a title compound as a white solid (21 mg, 23%).
¹H NMR (400 MHz, DMSO-d6) δ 8.82 (d, J = 8.2 Hz, 1H), 8.52 (s, 1H), 7.63 (s, 1H), 7.50 (d, J = 8.0 Hz, 1H), 7.39 (d, J = 7.6 Hz, 1H), 5.85 (t, J = 7.3 Hz, 2H), 5.59 (d, J = 5.7 Hz, 1H), 5.40 (s, 1H), 4.66 - 4.58 (m, 1H), 4.35 (s, 1H), 3.42 (dd, J = 10.5, 4.0 Hz, 1H), 3.15 - 3.03 (m, 1H), 3.01 -2.88 (m, 1H), 2.81 (dd, J = 11.0, 2.5 Hz, 1H), 2.62 -2.54 (m, 1H), 2.28 -2.02 (m, 1H); LCMS m/z 472 [M+H]+

### [Example 29] (1R)-1-[[2-chloro-9-[(2R,3R,4S)-3,4-dihydroxytetrahydrothiophen-2-yl]purin-6-yl]amino]indane-5-carbonitrile

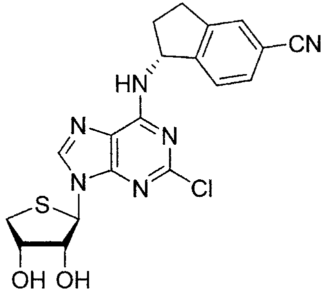

By using the same method as in Example 28, (2R,3R,4S)-2-(2,6-dichloro-9H-purin-9-yl)tetrahydrothiophene-3,4-diol (60 mg, 0.195 mmol), (1R)-1-amino-2,3-dihydro-1H-indene-5-carbonitrile (34 mg, 0.215 mmol), DIPEA (37 µL, 0.215 mmol) and 1,4-dioxane (2 mL) were used to obtain a title compound as an off-white solid (54 mg, 64%).
¹H NMR (400 MHz, DMSO-d6) δ 8.84 (d, J = 8.4 Hz, 1H), 8.52 (s, 1H), 7.75 (s, 1H), 7.60 (d, J = 7.9 Hz, 1H), 7.37 (d, J = 7.8 Hz, 1H), 5.84 (d, J = 6.7 Hz, 2H), 5.59 (d, J = 6.2 Hz, 1H), 5.41 (s, 1H), 4.61 (s, 1H), 4.35 (s, 1H), 3.42 (dd, J = 10.9, 3.9 Hz, 1H), 3.10 - 3.01 (m, 1H), 2.98 -2.86 (m, 1H), 2.80 (dd, J = 10.9, 2.5 Hz, 1H), 2.49 -2.40 (m, 1H), 2.25 -2.01 (m, 1H); LCMS m/z 429 [M+H]+

### [Example 30] Methyl (R)-1-((2-chloro-9-((2R,3R,4S)-3,4-dihydroxytetrahydrothiophen-2-yl)-9H-purin-6-yl)amino)-2,3-dihydro-1H-indene-4-carboxylate

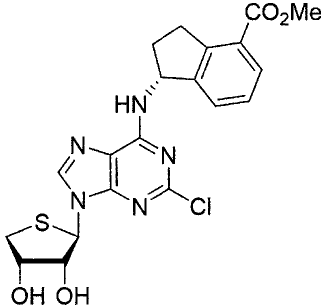

By using the same method as in Example 11, (2R,3R,4S)-2-(2,6-dichloro-9H-purin-9-yl)tetrahydrothiophene-3,4-diol (60 mg, 0.195 mmol), methyl (1R)-1-amino-2,3-dihydro-1H-indene-4-carboxylate hydrochloride (49 mg, 0.215 mmol), DMF (1.95 mL) and DIPEA (74 µL, 0.43 mmol) were used to obtain a title compound as an off-white solid (50 mg, 56%).
¹H NMR (400 MHz, DMSO-d6) δ 8.79 (d, J *=* 8.3 Hz, 1H), 8.51 (s, 1H), 7.82 (d, *J* = 7.1 Hz, 1H), 7.46 (d, *J =* 7.7 Hz, 1H), 7.31 (t, *J =* 7.7 Hz, 1H), 5.83 (d, *J =* 7.8 Hz, 2H), 5.59 (d, *J =* 7.0 Hz, 1H), 5.40 (d, *J =* 3.6 Hz, 1H), 4.66 - 4.57 (m, 1H), 4.35 (s, 1H), 3.85 (s, 3H), 3.49 - 3.36 (m, 2H), 3.15 - 3.02 (m, 1H), 2.81 (d, *J* = 10.9 Hz, 1H), 2.47 -2.43 (m, 1H), 2.23 - 1.96 (m, 1H); LCMS m/z 462 [M+H]+

### [Example 31] Methyl (R)-1-((2-chloro-9-((2R,3R,4S)-3,4-dihydroxytetrahydrothiophen-2-yl)-9H-purin-6-yl)amino)-2,3-dihydro-1H-indene-5-carboxylate

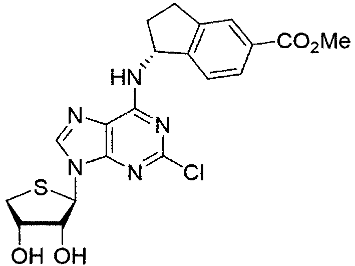

By using the same method as in Example 11, (2R,3R,4S)-2-(2,6-dichloro-9H-purin-9-yl)tetrahydrothiophene-3,4-diol (60 mg, 0.195 mmol), methyl (1R)-1-amino-2,3-dihydro-1H-indene-5-carboxylate hydrochloride (49 mg, 0.215 mmol), DMF (1.95 mL) and DIPEA (74 µL, 0.43 mmol) were used to obtain a title compound as a brown solid (72 mg, 80%).
¹H NMR (400 MHz, DMSO-d6) δ 8.81 (d, J = 8.6 Hz, 1H), 8.51 (s, 1H), 7.86 (s, 1H), 7.77 (d, J = 7.8 Hz, 1H), 7.30 (d, J = 7.7 Hz, 1H), 5.85 (t, J = 6.9 Hz, 2H), 5.59 (d, J = 5.8 Hz, 1H), 5.40 (d, J = 4.1 Hz, 1H), 4.62 (s, 1H), 4.35 (s, 1H), 3.84 (s, 3H), 3.42 (dd, J = 11.0, 3.9 Hz, 1H), 3.12 - 3.00 (m, 1H), 2.99 -2.85 (m, 1H), 2.81 (dd, J = 11.0, 2.3 Hz, 1H), 2.47 -2.44 (m, 1H), 2.27 -2.00 (m, 1H); LCMS m/z 462 [M+H]+

### [Example 32] (R)-1-((2-chloro-9-((2R,3R,4S)-3,4-dihydroxytetrahydrothiophen-2-yl)-9H-purin-6-yl)amino)-2,3-dihydro-1H-indene-4-carboxylic acid

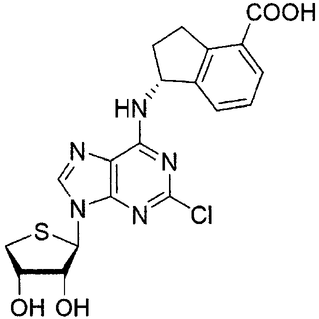

MeOH (0.65 mL) and 2 M NaOH (0.163 mL) were added to methyl (R)-1-((2-chloro-9-((2R,3R,4S)-3,4-dihydroxytetrahydrothiophen-2-yl)-9H-purin-6-yl)amino)-2,3-dihydro-1H-indene-4-carboxylate (30 mg, 0.065 mmol), and the mixture was stirred at room temperature for 2 hours. THF (0.5 mL) and 2 M NaOH (33 µL) were added, and the mixture was stirred for an additional 7 hours. 1 M HCl was added to the reaction mixture, and the resulting solid was filtered and dried to obtain a title compound as an orange solid (22 mg, 76%).
¹HNMR (400 MHz, DMSO-d6) δ 12.83 (s, 1H), 8.80 (d, J *=* 8.1 Hz, 1H), 8.51 (s, 1H), 7.83 (s, 1H), 7.75 (d, J = 8.1 Hz, 1H), 7.28 (d, J = 7.8 Hz, 1H), 5.84 (d, J = 7.0 Hz, 2H), 5.60 (s, 1H), 5.41 (s, 1H), 4.62 (s, 1H), 4.35 (s, 1H), 3.42 (dd, J = 10.9, 3.9 Hz, 1H), 3.06 (dd, J = 14.8, 8.3 Hz, 1H), 2.97 -2.85 (m, 1H), 2.81 (dd, J = 10.9, 2.5 Hz, 1H), 2.26 - 1.98 (m, 1H); LCMS m/z 448 [M+H]+

### [Example 33] (R)-1-((2-chloro-9-((2R,3R,4S)-3,4-dihydroxytetrahydrothiophen-2-yl)-9H-purin-6-yl)amino)-2,3-dihydro-1H-indene-5-carboxylic acid

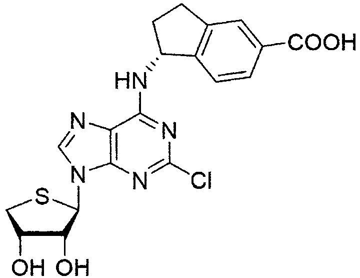

By using the same method as in Example 32, methyl (R)-1-((2-chloro-9-((2R,3R,4S)-3,4-dihydroxytetrahydrothiophen-2-yl)-9H-purin-6-yl)amino)-2,3-dihydro-1H-indene-5-carboxylate (30 mg, 0.065 mmol), MeOH (0.65 mL), THF (0.5 mL) and 2 M NaOH (0.163 mL) were used to obtain a title compound as a brown solid (21 mg, 72%).
¹H NMR (400 MHz, DMSO-d6) δ12.87 (s, 1H), 8.77 (d, J = 8.3 Hz, 1H), 8.50 (s, 1H), 7.81 (d, J = 7.7 Hz, 1H), 7.42 (d, J = 7.2 Hz, 1H), 7.27 (t, J = 7.7 Hz, 1H), 5.91 - 5.76 (m, 1H), 5.60 (s, 1H), 5.40 (s, 1H), 4.62 (s, 1H), 4.35 (s, 1H), 3.51 - 3.39 (m, 2H), 3.14 - 3.00 (m, 1H), 2.80 (dd, J = 10.8, 2.4 Hz, 1H), 2.18 - 1.97 (m, 1H); LCMS m/z 448 [M+H]+

### [Example 34] (R)-1-((2-chloro-9-((2R,3R,4S)-3,4-dihydroxytetrahydrothiophen-2-yl)-9H-purin-6-yl)amino)-2,3-dihydro-1H-indene-5-carboxamide

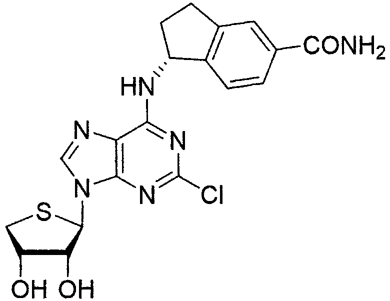

After dissolving (1R)-1-[[2-chloro-9-[(2R,3R,4S)-3,4-dihydroxytetrahydrothiophen-2-yl]purin-6-yl]amino]indane-5-carbonitrile (25 mg, 0.058 mmol) in DMSO (0.3 mL), K₂CO₃ (14.3 mg, 0.105 mmol) and H₂O₂ (35% in H₂O, 82.5 µL) were added. After stirring at room temperature for 2 hours, the mixture was diluted with EtOAc and washed with DW. The organic layer was washed with brine, dried with MgSO₄, filtered, and concentrated. The obtained residue was purified by column chromatography (0-3% MeOH/DCM) to obtain a title compound as a white solid (10 mg, 38%).
¹H NMR (400 MHz, DMSO-d6) δ 8.77 (d, J = 7.8 Hz, 1H), 8.51 (s, 1H), 7.90 (s, 1H), 7.76 (s, 1H), 7.67 (d, J = 8.0 Hz, 1H), 7.34 - 7.15 (m, 2H), 5.91 - 5.76 (m, 2H), 5.58 (d, J = 6.4 Hz, 1H), 5.39 (d, J = 4.0 Hz, 1H), 4.61 (s, 1H), 4.35 (s, 1H), 3.42 (dd, J = 10.7, 4.0 Hz, 1H), 3.17 (d, J = 5.2 Hz, 1H), 3.09 -2.97 (m, 1H), 2.95 -2.84 (m, 1H), 2.81 (dd, J = 11.0, 2.1 Hz, 1H), 2.26 - 1.98 (m, 1H); LCMS m/z 447 [M+H]⁺

### [Example 35] (2R,3R,4S)-2-(2-chloro-6-((1,2,3,4-tetrahydronaphthalen-2-yl)amino)-9H-purin-9-yl)tetrahydrothiophene-3,4-diol

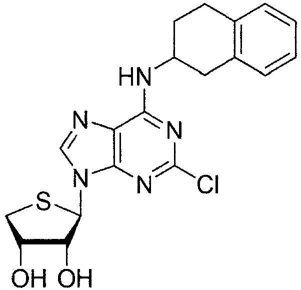

After dissolving (2R,3R,4S)-2-(2,6-dichloro-9H-purin-9-yl)tetrahydrothiophene-3,4-diol (60 mg, 0.195 mmol) and 1,2,3,4-tetrahydronaphthalen-2-amine hydrochloride (39.5 mg, 0.215 mmol) in DMF (1.95 mL), DIPEA (74 µL, 0.43 mmol) was added. After stirring at 50°C for 20 hours, DW was added, stirred for 30 minutes, filtered and dried. The obtained solid was dissolved in DCM, washed with DW, dried over MgSO₄, filtered and concentrated to obtain a title compound as an off-white solid (26 mg, 32%).
¹H NMR (400 MHz, DMSO-d6) δ 8.50 (s, 1H), 8.40 (d, J = 7.9 Hz, 1H), 7.16 - 7.02 (m, 4H), 5.82 (d, J = 7.2 Hz, 1H), 5.58 (d, J = 6.2 Hz, 1H), 5.39 (d, J = 3.8 Hz, 1H), 4.61 (s, 1H), 4.40 (s, 1H), 4.34 (s, 1H), 3.41 (dd, J *=* 10.8, 3.3 Hz, 1H), 3.17 -2.98 (m, 1H), 2.98 -2.83 (m, 3H), 2.80 (dd, J = 10.9, 2.3 Hz, 1H), 2.18 - 1.98 (m, 1H), 1.96 - 1.70 (m, 1H); LCMS m/z 418 [M+H]+

### [Example 36] (2R,3R,4S)-2-(2-chloro-6-(((S)-1,2,3,4-tetrahydronaphthalen-2-yl)amino)-9H-purin-9 yl)tetrahydrothiophene-3,4-diol

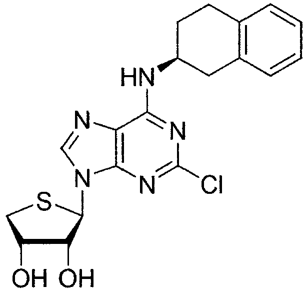

After dissolving (2R,3R,4S)-2-(2,6-dichloro-9H-purin-9-yl)tetrahydrothiophene-3,4-diol (60 mg, 0.195 mmol) and (S)-1,2,3,4-tetrahydronaphthalen-2-amine (31.7 mg, 0.215 mmol) in DCM (2 mL), DIPEA (37 µL, 0.215 mmol) was added. After stirring at room temperature for 41.5 hours and at 45°C for 4 hours, the reaction mixture was diluted with DCM and washed with DW. The organic layer was dried over MgSO₄, filtered and concentrated, and the obtained residue was purified by column chromatography (0-1% MeOH/DCM) to obtain a title compound as an orange solid (46 mg, 56%).
¹H NMR (400 MHz, DMSO-d6) δ 8.50 (s, 1H), 8.41 (d, J *=* 7.9 Hz, 1H), 7.10 (dd, J = 6.6, 3.4 Hz, 4H), 5.82 (d, J = 7.4 Hz, 1H), 5.58 (d, J = 6.2 Hz, 1H), 5.39 (d, J = 4.0 Hz, 1H), 4.60 (s, 1H), 4.41 (s, 1H), 4.34 (s, 1H), 3.41 (dd, J = 10.6, 3.7 Hz, 1H), 3.19 -2.98 (m, 1H), 2.97 -2.83 (m, 3H), 2.80 (dd, J *=* 11.0, 1.7 Hz, 1H), 2.18 -2.00 (m, 1H), 1.94 - 1.70 (m, 1H); LCMS m/z 418 [M+H]+

### [Example 37] (2R,3R,4S)-2-(2-chloro-6-(((R)-1,2,3,4-tetrahydronaphthalen-2-yl)amino)-9H-purin-9-yl)tetrahydrothiophene-3,4-diol

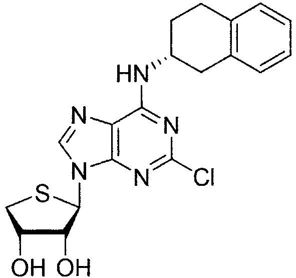

By using the same method as in Example 35, (2R,3R,4S)-2-(2,6-dichloro-9H-purin-9-yl)tetrahydrothiophene-3,4-diol (60 mg, 0.195 mmol), (R)-1,2,3,4-Tetrahydronaphthalen-2-amine (31.7 mg, 0.215 mmol), DMF (1.95 mL) and DIPEA (37 µL, 0.215 mmol) were used to obtain a title compound as a brown solid (56 mg, 69%).
¹H NMR (400 MHz, DMSO-d6) δ 8.50 (s, 1H), 8.40 (d, J = 7.8 Hz, 1H), 7.18 - 6.99 (m, 4H), 5.82 (d, J = 7.2 Hz, 1H), 5.58 (d, J = 6.3 Hz, 1H), 5.39 (d, J = 4.0 Hz, 1H), 4.60 (s, 1H), 4.48 - 4.26 (m, 2H), 3.41 (d, J = 9.8 Hz, 1H), 3.18 -2.99 (m, 1H), 2.99 -2.83 (m, 3H), 2.80 (d, J = 9.2 Hz, 1H), 2.19 - 1.96 (m, 1H), 1.94 - 1.71 (m, 1H); LCMS m/z 418 [M+H]+

### [Example 38] (2R,3R,4S)-2-(2-chloro-6-((1,2,3,4-tetrahydronaphthalen-1-yl)amino)-9H-purin-9-yl)tetrahydrothiophene-3,4-diol

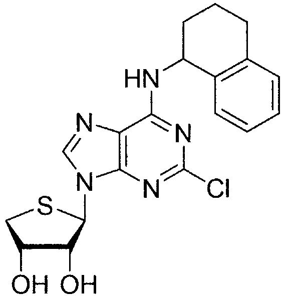

By using the same method as in Example 35, (2R,3R,4S)-2-(2,6-dichloro-9H-purin-9-yl)tetrahydrothiophene-3,4-diol (60 mg, 0.195 mmol), 1,2,3,4-tetrahydro-1-naphthylamine (31.7 mg, 0.215 mmol), DMF (1.95 mL) and DIPEA (37 µL, 0.215 mmol) were used to obtain a title compound as a yellow solid (30 mg, 37%).
¹H NMR (400 MHz, DMSO-d6) δ8.64 (d, J = 8.6 Hz, 1H), 8.49 (s, 1H), 7.22 - 7.03 (m, 4H), 5.83 (d, J = 7.0 Hz, 1H), 5.59 (d, J = 6.2 Hz, 1H), 5.50 (s, 1H), 5.40 (s, 1H), 4.61 (s, 1H), 4.35 (s, 1H), 3.42 (dd, J = 10.8, 3.5 Hz, 1H), 2.87 -2.70 (m, 3H), 2.12 - 1.69 (m, 4H); LCMS m/z 418 [M+H]+

### [Example 39] (2R,3R,4S)-2-(2-chloro-6-(((R)-1,2,3,4-tetrahydronaphthalen-1-yl)amino)-9H-purin-9-yl)tetrahydrothiophene-3,4-diol

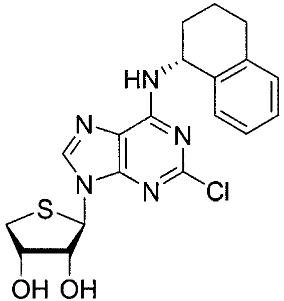

By using the same method as in Example 35, (2R,3R,4S)-2-(2,6-dichloro-9H-purin-9-yl)tetrahydrothiophene-3,4-diol (60 mg, 0.195 mmol), (R)-1,2,3,4-tetrahydronaphthalen-1-amine (31.7 mg, 0.215 mmol), DMF (1.95 mL) and DIPEA (37 µL, 0.215 mmol) were used to obtain a title compound as a yellow solid (44 mg, 54%).
¹H NMR (400 MHz, DMSO-d6) δ8.64 (d, J = 8.8 Hz, 1H), 8.57 - 8.42 (m, 1H), 7.23 - 7.01 (m, 4H), 5.83 (d, J = 7.2 Hz, 1H), 5.59 (d, J = 6.0 Hz, 1H), 5.50 (s, 1H), 5.39 (d, J = 3.8 Hz, 1H), 4.61 (s, 1H), 4.34 (s, 1H), 3.42 (dd, J = 10.8, 3.9 Hz, 1H), 2.87 -2.69 (m, 3H), 2.13 - 1.69 (m, 4H); LCMS m/z 418 [M+H]+

### [Example 40] (2R,3R,4S)-2-(2-chloro-6-(((S)-1,2,3,4-tetrahydronaphthalen-1-yl)amino)-9H-purin-9-yl)tetrahydrothiophene-3,4-diol

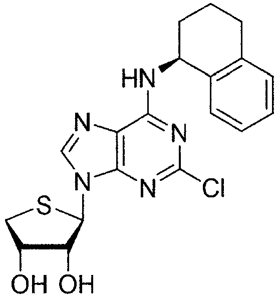

The reaction was performed in the same manner as in Example 35 by using (2R,3R,4S)-2-(2,6-dichloro-9H-purin-9-yl)tetrahydrothiophene-3,4-diol (60 mg, 0.195 mmol), (S)-1,2,3,4-tetrahydronaphthalen-1-amine (31.7 mg, 0.215 mmol), DMF (1.95 mL) and DIPEA (37 µL, 0.215 mmol). The obtained residue was purified by column chromatography (0-1% MeOH/DCM) to obtain a title compound as an off-white solid (45 mg, 55%).
¹H NMR (400 MHz, DMSO-d6) δ 8.65 (d, J *=* 8.9 Hz, 1H), 8.49 (s, 1H), 7.23 -7.02 (m, 4H), 5.83 (d, J = 7.3 Hz, 1H), 5.59 (d, J = 6.3 Hz, 1H), 5.51 (s, 1H), 5.40 (d, J = 3.9 Hz, 1H), 4.61 (s, 1H), 4.34 (s, 1H), 3.42 (dd, J *=* 10.7, 3.7 Hz, 1H), 2.87 -2.69 (m, 3H), 2.11 - 1.69 (m, 4H); LCMS m/z 418 [M+H]+

### [Example 41] (2R,3R,4S)-2-(6-(((R)-bicyclo[4.2.0]octa-1(6),2,4-trien-7-yl)amino)-2-chloro-9H-purin-9-yl)tetrahydrothiophene-3,4-diol

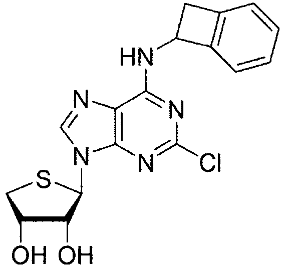

After dissolving (2R,3R,4S)-2-(2,6-dichloro-9H-purin-9-yl)tetrahydrothiophene-3,4-diol (100 mg, 0.326 mmol) and bicyclo[4.2.0]octa-1(6),2,4-trien-7-amine hydrochloride (58.4 mg, 0.375 mmol) in DCM (3 mL), DIPEA (128 µL, 0.75 mmol) was added. After stirring at room temperature for 24 hours, the reaction mixture was diluted with DCM and washed with DW. The organic layer was dried over MgSO₄, filtered and concentrated, and the resulting residue was purified by column chromatography (0-1% MeOH/DCM) to obtain a title compound as a white solid (90 mg, 71%).
¹H NMR (400 MHz, DMSO-d6) δ 9.09 (d, J = 6.0 Hz, 1H), 8.51 (s, 1H), 7.38 - 7.04 (m, 4H), 5.84 (d, J = 7.2 Hz, 1H), 5.59 (d, J = 5.2 Hz, 2H), 5.40 (d, J = 3.9 Hz, 1H), 4.61 (s, 1H), 4.34 (s, 1H), 3.63 (dd, J = 14.8, 4.5 Hz, 1H), 3.48 - 3.35 (m, 2H), 2.81 (d, J = 11.5 Hz, 1H); LCMS m/z 390 [M+H]+

### [Example 42] (2R,3R,4S)-2-(2-chloro-6-((6,7,8,9-tetrahydro-5H-benzo[7]annulen-5-yl)amino)-9H -purin-9-yl)tetrahydrothiophene-3,4-diol

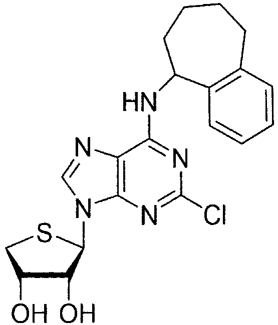

By using the same method as in Example 41, (2R,3R,4S)-2-(2,6-dichloro-9H-purin-9-yl)tetrahydrothiophene-3,4-diol (60 mg, 0.195 mmol), 6,7,8,9-tetrahydro-5H-benzo[7]annulen-5-amine (34.6 mg, 0.215 mmol), DCM (2 mL) and DIPEA (37 µL, 0.215 mmol) were used to obtain a title compound as a white solid (42 mg, 50%).
¹H NMR (400 MHz, DMSO-d6) δ 8.93 (d, J = 8.4 Hz, 1H), 8.55 (d, J = 3.9 Hz, 1H), 7.35 - 6.98 (m, 4H), 5.88 - 5.76 (m, 1H), 5.58 (d, J = 5.9 Hz, 1H), 5.49 - 5.30 (m, 2H), 4.62 (s, 1H), 4.35 (s, 1H), 3.42 (d, J = 11.0 Hz, 1H), 3.03 -2.69 (m, 3H), 2.15 - 1.55 (m, 5H), 1.27 (s, 1H); LCMS m/z 432 [M+H]+

### [Example 43] (2R,3R,4S)-2-(2-chloro-6-((6,7,8,9-tetrahydro-5H-benzo[7]annulen-7-yl)amino)-9H -purin-9-yl)tetrahydrothiophene-3,4-diol

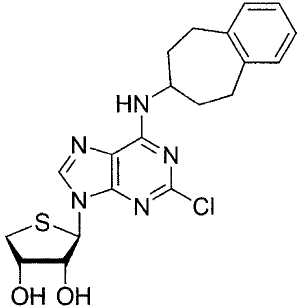

By using the same method as in Example 41, (2R,3R,4S)-2-(2,6-dichloro-9H-purin-9-yl)tetrahydrothiophene-3,4-diol (60 mg, 0.195 mmol), 6,7,8,9-Tetrahydro-5H-benzocyclohepten-7-ylamine Hydrochloride (42.5 mg, 0.215 mmol), DCM (2 mL) and DIPEA (74 µL, 0.43 mmol) were used to obtain a title compound as a white solid (38 mg, 45%).
¹H NMR (400 MHz, DMSO-d6) δ8.46 (s, 1H), 8.29 (d, J = 8.8 Hz, 1H), 7.24 - 7.00 (m, 4H), 5.81 (d, J = 7.2 Hz, 1H), 5.57 (d, J = 6.1 Hz, 1H), 5.38 (d, J = 3.5 Hz, 1H), 4.59 (s, 1H), 4.33 (s, 2H), 3.41 (dd, J = 11.2, 3.8 Hz, 1H), 2.89 -2.71 (m, 5H), 2.08 (s, 2H), 1.54 - 1.32 (m, 2H); LCMS m/z 432 [M+H]+

### [Example 44] (2R,3R,4S)-2-(6-(((R)-2,3-dihydro-1H-inden-1-yl)amino)-2-iodo-9H-purin-9-yl)tetrahydrothiophene-3,4-diol

### Step 1: (2R,3R,4S)-2-(6-chloro-2-iodo-9H-purin-9-yl)tetrahydrothiophene-3,4-diol

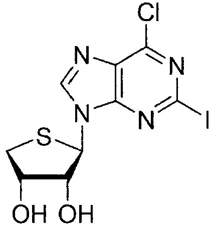

By using the same method as in Preparation Example 6, 6-chloro-9-((3aR,4R,6aS)-2,2-dimethyltetrahydrothieno[3,4-d][1,3]dioxol-4-yl)-2-iodo-9H-purine (150 mg, 0.342 mmol) and 80% HCOOH (6.8 mL) were used to obtain a title compound as a white solid (57 mg, 42%).
¹H NMR (400 MHz, DMSO-d6) δ 8.99 (s, 1H), 5.91 (d, J = 7.3 Hz, 1H), 5.64 (d, J = 5.8 Hz, 1H), 5.45 (d, J = 4.0 Hz, 1H), 4.61 (td, J = 6.7, 3.2 Hz, 1H), 4.39 - 4.31 (m, 1H), 3.45 (dd, J = 10.8, 3.9 Hz, 1H), 2.82 (dd, J = 10.8, 2.4 Hz, 1H); LCMS m/z 399 [M+H]+

### Step 2: (2R,3R,4S)-2-(6-(((R)-2,3-dihydro-1H-inden-1-yl)amino)-2-iodo-9H-purin-9-yl)tetrahydrothiophene-3,4-diol

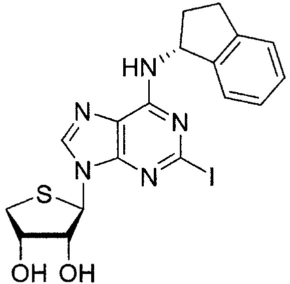

After dissolving (2R,3R,4S)-2-(6-chloro-2-iodo-9H-purin-9-yl)tetrahydrothiophene-3,4-diol (55 mg, 0.138 mmol) and (R)-(-)-1-aminoindane (20.2 mg, 0.152 mmol) in DMF (1.38 mL), DIPEA (26 µL, 0.152 mmol) was added. The mixture was stirred at room temperature for 63.5 hours, and then, DW was added and stirred for 30 minutes. The resulting solid was filtered and dissolved in 0.5 mL of DCM, 3 mL of hexane was added, and it was stirred at room temperature for 30 minutes. The resulting solid was filtered and dried to obtain a title compound as a yellow solid (42.6 mg, 62%).
¹H NMR (400 MHz, DMSO-d6) δ8.54 (d, J = 7.8 Hz, 1H), 8.42 (s, 1H), 7.33 - 7.07 (m, 4H), 5.82 (d, J = 7.3 Hz, 1H), 5.76 (d, J = 7.2 Hz, 1H), 5.58 (d, J = 5.9 Hz, 1H), 5.40 (s, 1H), 4.65 - 4.54 (m, 1H), 4.34 (s, 1H), 3.41 (dd, J = 10.9, 3.4 Hz, 1H), 3.06 -2.96 (m, 1H), 2.90 -2.78 (m, 2H), 2.47 -2.37 (m, 1H), 2.21 - 1.95 (m, 1H); LCMS m/z 496 [M+H]⁺

### [Example 45] (2R,3R,4S)-2-(6-(((R)-2,3-dihydro-1H-inden-1-yl)amino)-9H-purin-9-yl)tetrahydrothiophene-3,4-diol

### Step 1: (2R,3R,4S)-2-(6-chloro-9H-purin-9-yl)tetrahydrothiophene-3,4-diol

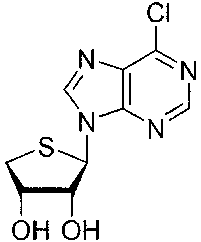

By using the same method as in Preparation Example 6, 6-chloro-9-((3aR,4R,6aS)-2,2-dimethyltetrahydrothieno[3,4-d][1,3]dioxol-4-yl)-9H-purine (510 mg, 1.63 mmol) and 80% HCOOH (32 mL) were used to obtain a title compound as a white solid (180 mg, 40%).
LCMS m/z 273 [M+H]⁺

### Step 2: (2R,3R,4S)-2-(6-(((R)-2,3-dihydro-1H-inden-1-yl)amino)-9H-purin-9-yl)tetrahydrothiophene-3,4-diol

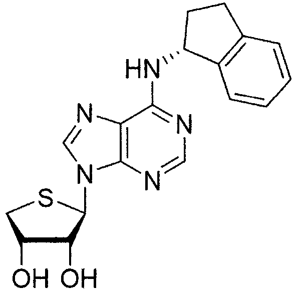

After dissolving (2R,3R,4S)-2-(6-chloro-9H-purin-9-yl)tetrahydrothiophene-3,4-diol (25 mg, 0.092 mmol) and (R)-(-)-1-aminoindane (13.4 mg, 0.101 mmol) in DMF (1 mL), DIPEA (17.1 µL, 0.101 mmol) was added. After stirring at room temperature for 61.5 hours, DW was added to the reaction mixture, and it was extracted with EA. The organic layer was dried over MgSO₄, filtered and concentrated, and the residue was purified by column chromatography (0-1% MeOH/DCM) to obtain a title compound as an off-white solid (6 mg, 18%).
¹H NMR (400 MHz, DMSO-d6) δ 8.44 (s, 1H), 8.28 (s, 1H), 8.08 (s, 1H), 7.26 (d, J = 7.5 Hz, 1H), 7.23 - 7.08 (m, 3H), 5.93 (d, J = 7.1 Hz, 2H), 5.55 (d, J = 6.2 Hz, 1H), 5.36 (d, J = 3.9 Hz, 1H), 4.70 (dd, J = 9.1, 6.9 Hz, 1H), 4.43 - 4.30 (m, 1H), 3.42 (dd, J = 10.8, 4.0 Hz, 1H), 3.01 (dd, J = 15.5, 8.9 Hz, 1H), 2.90 -2.76 (m, 2H), 2.47 - 2.40 (m, 1H), 2.20 -2.04 (m, 1H); LCMS m/z 370 [M+H]⁺

### Test Example 1. Test for inhibition of endothelial cell metastasis by radiation using immunofluorescence staining

Test substances (Examples 1 to 45) were treated with a 10 mM DMSO solution at a ratio of 1 µL per 1 mL of cell culture medium to a final concentration of 10 uM, and the test substances were treated 1 hour before irradiation on human vascular endothelial cell lines (Promocell, Germany), and then, it was irradiated at an intensity of 10 Gy. 72 hours after irradiation, the human vascular endothelial cell lines were fixed with 10% formalin, and the experiment was conducted according to the manufacturer's method using phalloidin (Invitrogen, USA) to confirm the cytoskeleton filaments that increase during fibrosis. Phalloidin is a protein that binds to the actin of cytoskeleton filaments, and phalloidin conjugated with FITC fluorescent substance made the cytoskeleton filaments appear green when observed under a microscope.

After treatment with the test substance, the area, length and density of phalloidin were measured by using a confocal microscope, and the relative ratio was expressed with the irradiated group (IR group) that was irradiated with 10 Gy alone as 100% (Table 1).

**[Table 1]**

| **Example** | **Palloidin Area** | **Palloidin Length** | **Palloidin Intensity** |
|---|---|---|---|
| 1 | ++ | + | ++ |
| 2 | + | + | + |
| 3 | + | + | + |
| 4 | ++ | ++ | ++ |
| 5 | ++ | ++ | ++ |
| 6 | +++ | ++⁺ | +++ |
| 7 | + | + | + |
| 8 | +++ | +++ | +++ |
| 9 | ++ | ++ | ++ |
| 10 | + | + | + |
| 11 | +++ | +++ | +++ |
| 12 | + | + | + |
| 13 | ++ | ++ | ++ |
| 14 | ++ | ++ | ++ |
| 15 | ++++ | ++++ | ++++ |
| 16 | ++++ | ++++ | ++++ |
| 17 | ++++ | ++++ | ++++ |
| 18 | ++++ | ++++ | ++++ |
| 19 | +++ | +++ | +++ |
| 20 | ++++ | ++++ | +++ |
| 21 | +++ | +++ | +++ |
| 22 | ++++ | + + + + | ++++ |
| 23 | + | + | + |
| 24 | ++ | ++ | ++ |
| 25 | + | + | + |
| 26 | +++ | ++ | ++ |
| 27 | + | + | + |
| 28 | ++ | + | ++ |
| 29 | ++ | + | ++ |
| 30 | + | + | + |
| 31 | + | + | + |
| 32 | + | + | + |
| 33 | + | + | + |
| 34 | + | + + | + |
| 35 | + | + | + |
| 36 | + | + | + |
| 37 | ++ | + | ++ |
| 38 | + | + | + |
| 39 | + | + | + |
| 40 | + | + | + |
| 41 | + | + | ++ |
| 42 | + | + | + |
| 43 | + | + | + |
| 44 | +++ | +++ | +++ |
| 45 | + | + | + |
| No IR | 4.7% | 3.1% | 3.2% |
| IR | 99.2% | 100.0% | 100.0010 |

| | | | |
|---|---|---|---|
| ++++: <20% +++: 20%~50% ++: 50%~80% +: >80% | | | |

As can be confirmed in Table 1, in the group that was not irradiated (No IR group), phalloidin was not observed much, but in the irradiated group (IR group), phalloidin increased significantly, and when it was treated with the compound according to the present invention, although there was a difference in the degree, it was shown that all of them suppressed an increase in phalloidin due to irradiation, confirming that the compound according to the present invention can reduce the transition of endothelial cells into mesenchymal cells (EndMT) due to irradiation.

Through the above results, it was found that when the compound according to the present invention is treated, it can exhibit an EndMT inhibitory effect and a preventive or therapeutic effect for diseases associated with EndMT activity (e.g., pulmonary disease).

### National Research and Development Project that Supported This Invention

[Project Identification Number] 1711187521
[Project Number] NRF-2020M2D9A2093963
[Name of Ministry] Ministry of Science and ICT
[Name of Project Management (Specialized) Institution] National Research Foundation of Korea
[Title of Research Project] Nuclear Energy Research and Development Project - Advanced Radiation Fusion Therapy Technology Development Project
[Title of Research Task] Development of Radiation-Based Pulmonary Fibrosis Targeted Treatment Based on Leading Technology
[Contribution Ratio] 1/1
[Name of Project Executing Institution] Korea Institute of Radiological and Medical Sciences, Nextgen Bioscience Co., Ltd., Yonsei University
[Research Period] July 31, 2020 to December 31, 2023

## Claims

1. A compound represented by Chemical Formula (1) below, an optical isomer thereof, a racemic mixture thereof, a hydrate thereof, a solvate thereof or a pharmaceutically acceptable salt thereof: 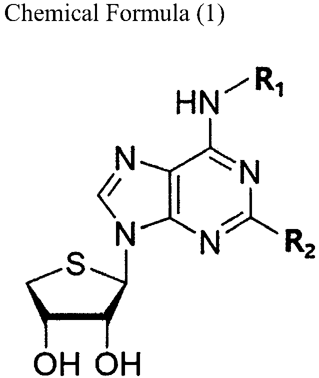
wherein R₁ is a fused bicyclic ring in which an aryl ring and a non-aromatic cycloalkyl ring are fused,
wherein the fused bicyclic ring is substituted or unsubstituted and
wherein R₂ is hydrogen (H), halogen (X) or an alkynyl group.

2. The compound, optical isomer thereof, racemic mixture thereof, hydrate thereof, solvate thereof or pharmaceutically acceptable salt thereof according to claim 1, wherein the fused bicyclic ring is benzocycloalkyl.

3. The compound, optical isomer thereof, racemic mixture thereof, hydrate thereof, solvate thereof or pharmaceutically acceptable salt thereof according to claim 1, wherein a substitution in the fused bicyclic ring is that any one or more hydrogens of an aryl ring and a non-aromatic cycloalkyl ring are independently substituted with C₁₋₆ alkoxy, halogen, C₁₋₆ alkyl, CF₃, CN, CO₂Me, CONH₂ or COOH.

4. The compound, optical isomer thereof, racemic mixture thereof, hydrate thereof, solvate thereof or pharmaceutically acceptable salt thereof according to claim 1, wherein the alkynyl group is a ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group or a 1,1-dimethyl-2-butynyl group.

5. The compound, optical isomer thereof, racemic mixture thereof, hydrate thereof, solvate thereof or pharmaceutically acceptable salt thereof according to claim 1, wherein the compound is selected from the following group:
(1) (2R,3R,4S)-2-[6-[[(1R)-indan-1-yl]amino]-2-prop-1-ynyl-purin-9-yl]tetrahydrothiophene-3,4-diol;
(2) (2R,3R,4S)-2-[6-[[(1S)-indan-1-yl]amino]-2-prop-1-ynyl-purin-9-yl]tetrahydrothiophene-3,4-diol;
(3) (2R,3R,4S)-2-[2-but-1-ynyl-6-[[(1R)-indan-1-yl]amino]purin-9-yl]tetrahydrothiophene-3,4-diol;
(4) (2R,3R,45)-2-[2-but-1-ynyl-6-[[(1S)-indan-1-yl]amino]purin-9-yl]tetrahydrothiophene-3,4-diol;
(5) (2R,3R,4S)-2-[6-[[(1R)-indan-1-yl]amino]-2-pent-1-ynyl-purin-9-yl]tetrahydrothiophene-3,4-diol;
(6) (2R,3R,4S)-2-[6-[[(1S)-indan-1-yl]amino]-2-pent-1-ynyl-purin-9-yl]tetrahydrothiophene-3,4-diol;
(7) (2R,3R,4S)-2-[2-chloro-6-(indan-2-ylamino)purin-9-yl]tetrahydrothiophene-3,4-diol;
(8) (2R,3R,4S)-2-[2-chloro-6-(indan-1-ylamino)purin-9-yl]tetrahydrothiophene-3,4-diol;
(9) (2R,3R,4S)-2-[2-chloro-6-[[(1R)-indan-1-yl]amino]purin-9-yl]tetrahydrothiophene-3,4-diol;
(10) (2R,3R,4S)-2-[2-chloro-6-[[(1S)-indan-1-yl]amino]purin-9-yl]tetrahydrothiophene-3,4-diol;
(11) (2R,3R,4S)-2-[2-chloro-6-[[(1R)-4-methoxyindan-1-yl]amino]purin-9-yl]tetrahydrothiophene-3,4-diol;
(12) (2R,3R,4S)-2-[2-chloro-6-[[(1R)-5-methoxyindan-1-yl]amino]purin-9-yl]tetrahydrothiophene-3,4-diol;
(13) (2R,3R,4S)-2-[2-chloro-6-[[(1R)-6-methoxyindan-1-yl]amino]purin-9-yl]tetrahydrothiophene-3,4-diol;
(14) (2R,3R,4S)-2-[2-chloro-6-[[(1R)-7-methoxyindan-1-yl]amino]purin-9-yl]tetrahydrothiophene-3,4-diol;
(15) (2R,3R,4S)-2-[2-chloro-6-[[(1R)-4-fluoroindan-1-yl]amino]purin-9-yl]tetrahydrothiophene-3,4-diol;
(16) (2R,3R,4S)-2-[2-chloro-6-[[(1R)-5-fluoroindan-1-yl]amino]purin-9-yl]tetrahydrothiophene-3,4-diol;
(17) (2R,3R,4S)-2-[2-chloro-6-[[(1R)-4-chloroindan-1-yl]amino]purin-9-yl]tetrahydrothiophene-3,4-diol;
(18) (2R,3R,4S)-2-[2-chloro-6-[[(1R)-5-chloroindan-1-yl]amino]purin-9-yl]tetrahydrothiophene-3,4-diol;
(19) (2R,3R,4S)-2-[2-chloro-6-[[(1R)-4-bromoindan-1-yl]amino]purin-9-yl]tetrahydrothiophene-3,4-diol;
(20) (2R,3R,4S)-2-[2-chloro-6-[[(1R)-5-bromoindan-1-yl]amino]purin-9-yl]tetrahydrothiophene-3,4-diol;
(21) (2R,3R,4S)-2-[2-chloro-6-[[(1R)-6-bromoindan-1-yl]amino]purin-9-yl]tetrahydrothiophene-3,4-diol;
(22) (2R,3R,4S)-2-[2-chloro-6-[[(1R)-4-methylindan-1-yl]amino]purin-9-yl]tetrahydrothiophene-3,4-diol;
(23) (2R,3R,4S)-2-[2-chloro-6-[[(1R)-4-(trifluoromethyl)indan-1-yl]amino]purin-9-yl]tetrahydrothiophene-3,4-diol;
(24) (1R)-1-[[2-chloro-9-[(2R,3R,4S)-3,4-dihydroxytetrahydrothiophen-2-yl]purin-6-yl]amino]indane-4-carbonitrile;
(25) (R)-1-((2-chloro-9-((2R,3R,4S)-3,4-dihydroxytetrahydrothiophen-2-yl)-9H-purin-6-yl)amino)-2,3-dihydro-1H-indene-4-carboxamide;
(26) (2R,3R,4S)-2-[2-chloro-6-[[(1R)-7-bromoindan-1-yl]amino]purin-9-yl]tetrahydrothiophene-3,4-diol;
(27) (2R,3R,4S)-2-[2-chloro-6-[[(1R)-5-methylindan-1-yl]amino]purin-9-yl]tetrahydrothiophene-3,4-diol;
(28) (2R,3R,4S)-2-[2-chloro-6-[[(1R)-5-(trifluoromethyl)indan-1-yl]amino]purin-9-yl]tetrahydrothiophene-3,4-diol);
(29) (1R)-1-[[2-chloro-9-[(2R,3R,4S)-3,4-dihydroxytetrahydrothiophen-2-yl]purin-6-yl]amino]indane-5-carbonitrile;
(30) Methyl (R)-1-((2-chloro-9-((2R,3R,4S)-3,4-dihydroxytetrahydrothiophen-2-yl)-9H-purin-6-yl)amino)-2,3-dihydro-1H-indene-4-carboxylate;
(31) Methyl (R)-1-((2-chloro-9-((2R,3R,4S)-3,4-dihydroxytetrahydrothiophen-2-yl)-9H-purin-6-yl)amino)-2,3-dihydro-1H-indene-5-carboxylate;
(32) (R)-1-((2-chloro-9-((2R,3R,4S)-3,4-dihydroxytetrahydrothiophen-2-yl)-9H-purin-6-yl)amino)-2,3-dihydro-1H-indene-4-carboxylic acid;
(33) (R)-1-((2-chloro-9-((2R,3R,4S)-3,4-dihydroxytetrahydrothiophen-2-yl)-9H-purin-6-yl)amino)-2,3-dihydro-1H-indene-5-carboxylic acid;
(34) (R)-1-((2-chloro-9-((2R,3R,4S)-3,4-dihydroxytetrahydrothiophen-2-yl)-9H-purin-6-yl)amino)-2,3-dihydro-1H-indene-5-carboxamide;
(35) (2R,3R,4S)-2-(2-chloro-6-((1,2,3,4-tetrahydronaphthalen-2-yl)amino)-9H-purin-9-yl)tetrahydrothiophene-3,4-diol;
(36) (2R,3R,4S)-2-(2-chloro-6-(((S)-1,2,3,4-tetrahydronaphthalen-2-yl)amino)-9H-purin-9-yl)tetrahydrothiophene-3,4-diol;
(37) (2R,3R,4S)-2-(2-chloro-6-(((R)-1,2,3,4-tetrahydronaphthalen-2-yl)amino)-9H-purin-9-yl)tetrahydrothiophene-3,4-diol;
(38) (2R,3R,4S)-2-(2-chloro-6-((1,2,3,4-tetrahydronaphthalen-1-yl)amino)-9H-purin-9-yl)tetrahydrothiophene-3,4-diol;
(39) (2R,3R,4S)-2-(2-chloro-6-(((R)-1,2,3,4-tetrahydronaphthalen-1-yl)amino)-9H-purin-9-yl)tetrahydrothiophene-3,4-diol;
(40) (2R,3R,4S)-2-(2-chloro-6-(((S)-1,2,3,4-tetrahydronaphthalen-1-yl)amino)-9H-purin-9-yl)tetrahydrothiophene-3,4-diol);
(41) (2R,3R,4S)-2-(6-(((R)-bicyclo[4.2.0]octa-1(6),2,4-trien-7-yl)amino)-2-chloro-9H-purin-9-yl)tetrahydrothiophene-3,4-diol;
(42) (2R,3R,4S)-2-(2-chloro-6-((6,7,8,9-tetrahydro-5H-benzo[7]annulen-5-yl)amino)-9H-purin-9-yl)tetrahydrothiophene-3,4-diol;
(43) (2R,3R,4S)-2-(2-chloro-6-((6,7,8,9-tetrahydro-5H-benzo[7]annulen-7-yl)amino)-9H-purin-9-yl)tetrahydrothiophene-3,4-diol;
(44) (2R,3R,4S)-2-(6-(((R)-2,3-dihydro-1H-inden-1-yl)amino)-2-iodo-9H-purin-9-yl)tetrahydrothiophene-3,4-diol; and
(45) (2R,3R,4S)-2-(6-(((R)-2,3-dihydro-1H-inden-1-yl)amino)-9H-purin-9-yl)tetrahydrothiophene-3,4-diol.

6. The compound, optical isomer thereof, racemic mixture thereof, hydrate thereof, solvate thereof or pharmaceutically acceptable salt thereof according to claim 1, wherein the compound, optical isomer thereof, racemic mixture thereof, hydrate thereof, solvate thereof or pharmaceutically acceptable salt thereof inhibits endothelial-to-mesenchymal transition (EndMT).

7. A pharmaceutical composition for preventing or treating a pulmonary disease, comprising the compound, optical isomer thereof, racemic mixture thereof, hydrate thereof, solvate thereof or pharmaceutically acceptable salt thereof according to any one of claims 1 to 6 as an active ingredient.

8. The pharmaceutical composition of claim 7, wherein the pulmonary disease is pneumonia, pulmonary fibrosis or lung cancer.

9. The pharmaceutical composition of claim 7, wherein the pulmonary disease is a radiation-induced pulmonary disease.

10. The pharmaceutical composition of claim 7, wherein the pharmaceutical composition is used in conjunction with radiation therapy.

11. The pharmaceutical composition of claim 10, wherein the pharmaceutical composition is administered before and/or after radiation therapy.

12. An endothelial-to-mesenchymal transition (EndMT) inhibitor, comprising the compound, optical isomer thereof, racemic mixture thereof, hydrate thereof, solvate thereof or salt thereof according to any one of claims 1 to 6 as an active ingredient.

13. The endothelial-to-mesenchymal transition (EndMT) inhibitor, wherein the compound, optical isomer thereof, racemic mixture thereof, hydrate thereof, solvate thereof or salt thereof inhibits actin stress fiber formation in endothelial cells.
